# EUROPEAN PATENT APPLICATION

(11) **EP 3 524 500 A1**
(43) Date of publication of application: **14.08.2019**
(21) Application number: 17868764.6
(22) Date of filing: 24.07.2017
(51) Int. Cl.: B62J 99/00, B60R 16/037

(54) **VEHICLE ANIMACY PERCEPTION PROMOTION SYSTEM, VEHICLE, VEHICLE ANIMACY PERCEPTION PROMOTION PROGRAM, AND CONTROL METHOD**

(30) Priority: 08.11.2016 JP 2016218306
(71) Applicant: Yamaha Hatsudoki Kabushiki Kaisha, Iwata-shi, Shizuoka 438-8501 (JP)
(72) Inventor: SUEGAMI, Takashi, Iwata-shi Shizuoka 438-8501 (JP); DAIMOTO, Hiroshi, Iwata-shi Shizuoka 438-8501 (JP); TANAKA, Soichiro, Iwata-shi Shizuoka 438-8501 (JP); HARA, Ioki, Iwata-shi Shizuoka 438-8501 (JP); KAWASHIMA, Masaya, Iwata-shi Shizuoka 438-8501 (JP)
(74) Representative: Stöckeler, Ferdinand
(86) International application number: PCT/JP2017/026727
(87) International publication number: WO 2018/087962

(57) **Abstract**

The vehicle animacy perception promotion system 90 includes an occupant pressure detection unit 42 that detects action of an occupant on a vehicle applying pressure to the vehicle using his/her muscle strength, two contact units 8R, 8L that make contact with different body parts of the occupant, an actuator 6, an instruction unit 52, and a driving unit 53. The actuator 6 applies force to at least one of the two contact units such that the two contact units sandwich a body part of the occupant located between the two contact units to apply pressure to the body part. The force contains a component that brings the two contact units closer to each other in the direction connecting parts of the two contact units contacting the body part. The instruction unit 52 determines the force to be applied by the actuator to at least one of the two contact units based on the action detected by the occupant pressure detection unit in order to promote the occupant to perceive animacy in the vehicle.

## Description

### TECHNICAL FIELD

The present teaching relates to a vehicle having a function of allowing an occupant to perceive animacy in the vehicle to change the psychological state or physiological state of the occupant.

### BACKGROUND ART

Japanese Patent Application Publication No. 4-250171 (Patent Document 1) discloses a technology of a human-machine system, which is a man-machine system that appeals to the sensibility of a driver in association with a vehicle. This human-machine system includes a human state detection unit that presumes the physiological and psychological states of a driver, a stimulation control unit that determines a pattern of presenting human-like reactions to the driver, and a sensible stimulus generation unit that generates a stimulus for the driver based on a command from the stimulation control unit. More specifically, state quantities representing the level of irritation and surprise of the driver are calculated from detection results of a finger plethysmograph detection device. In accordance with parameters based on the state quantities, a vibration generator supporting a driver's seat is driven. The vibration to shake the seat stimulates the driver. Consequently, the driver can drive the vehicle comfortably.

Japanese Patent No. 5256826 (Patent Document 2) discloses a driving posture adjustment device that adjusts the relative position between a steering unit and a seat in a vehicle based on loads acting on the steering unit and lateral acceleration generated in the vehicle. In consideration of the influence of the load applied to the steering unit due to the lateral acceleration, the relative position between the steering unit and seat can be adjusted appropriately.

### CITATION LIST

### PATENT DOCUMENT

Patent Document 1: Japanese Patent Application Publication No. 4-250171
Patent Document 2: Japanese Patent No. 5256826

### SUMMARY OF INVENTION

### TECHNICAL PROBLEM

The human-machine system in the aforementioned related art artificially humanizes a machine by detecting human sensibility, and applying or not applying a machine's mechanical function to the human sensibility. In addition, the driving posture adjustment device in the aforementioned related art appropriately adjusts the relative position between the steering unit and seat based on an input of the steering unit during traveling. The present teaching discloses a vehicle allowing an occupant to perceive animacy in the vehicle by detecting the occupant's action and providing a stimulus to the occupant in a different manner from the aforementioned related art to thereby change the psychological state or physiological state of the occupant.

### SOLUTION TO PROBLEM AND ADVANTAGEOUS EFFECTS OF INVENTION

The human brain feels pleasure and excitement when it experiences a novel sensation or receives a novel stimulus. Improvement of traveling performance and drivability of a vehicle can promote the novel sensation or stimulus to an occupant, thereby enhancing the level of the occupant's satisfaction with the vehicle. On the other hand, the occupant may become accustomed to such sensations and stimulus if the occupant is repeatedly exposed to such sensations and stimulus. Therefore, the occupant who is accustomed to the traveling performance and drivability of the vehicle becomes bored of the vehicle. Consequently, it will be hard for the occupant to feel excitement. The occupant's satisfaction with the vehicle may sometimes decrease eventually.

To improve the occupant's satisfaction with the vehicle irrespective of an improvement of the traveling performance and drivability of the vehicle, the inventors conducted research on the development of a vehicle enabling the occupant to perceive animacy in the vehicle. As a result, the inventors found that the occupant perceives animacy in the vehicle when the occupant recognizes that a vehicle exists as an interactive object, or, in short, when the occupant feels the presence of the vehicle.

To this end, the inventors studied interaction manners between an occupant and a vehicle. Firstly, the inventors minutely examined emotional changes of the occupant on the vehicle and his/her behaviors associated therewith. The examination results show that the occupant's emotional changes often appear as an approach movement of the occupant's separate body parts apart from each other. For instance, many occupants in an excited mood tend to move their fingers and palms closer to each other to make a grasping shape, to move their right knees and left knees, or to move their bellies and thighs. In addition, the inventors found that the approach movement of the occupant's separate body parts is closely related to the occupant's action of applying pressure to the vehicle.

The inventors found that the occupant's emotional changes can be detected by detecting the approach movement of the occupant's separate body parts or by detecting the occupant's action of applying pressure to the vehicle. Furthermore, the inventors arrived at a configuration to achieve interaction between the occupant and vehicle by causing the vehicle to react to the detected movement or action of the occupant in imitation of the movement or action. In other words, the inventors arrived at a configuration in which the vehicle applies force to two contact units, which are apart from each other and are part of the vehicle in contact with the occupant's body, so as to bring the contact units closer to each other when the vehicle detects the occupant's action. It is also found that this configuration can have the occupant perceive animacy in the vehicle, and change the psychological state or physiological state of the occupant. Based on the above-described findings the inventors arrived at the following vehicle configurations.

### (First Configuration)

A vehicle animacy perception promotion system in the first configuration according to one aspect of the present teaching includes: an occupant pressure detection unit detecting action of an occupant, who is on a vehicle, applying pressure to the vehicle using his/her muscle strength; two contact units making contact with different body parts of the occupant; an actuator applying force to at least one of the two contact units such that the two contact units sandwich a body part of the occupant located between the two contact units to apply pressure to the body part, the force containing a component that brings the two contact units closer to each other in the direction connecting parts of the two contact units contacting the body part; an instruction unit determining the force to be applied by the actuator to at least one of the two contact units based on the action detected by the occupant pressure detection unit to promote the occupant to perceive animacy in the vehicle; and a driving unit driving the actuator upon receipt of an instruction indicating the force determined by the instruction unit from the instruction unit.

In the first configuration, the occupant pressure detection unit detects the action of the occupant applying pressure to the vehicle. For instance, the occupant pressure detection unit detects action made by an occupant to the vehicle, for example, grasping, embracing, sandwiching, and caressing the vehicle. Emotional change of the occupant can be detected by detecting the action. The instruction unit and driving unit apply force to the occupant's body part in contact with the vehicle based on the action detected by the occupant pressure detection unit. The force can provide the occupant with a sense of touch corresponding to the emotional change of the occupant. Then the actuator applies force, which contains a component that brings the two contact units closer to each other in the direction connecting the two contact units in contact with the different body parts of the occupant, to at least one of the two contact units. At this point, the two contact units apply pressure to the body parts of the occupant to sandwich a body part of the occupant between the two contact units. Therefore, the occupant can feel as if his/her two separate body parts are wrapped, embraced, grasped, or sandwiched by the vehicle. Thus, the occupant can feel as if he/she is wrapped or embraced by the vehicle in sync with his/her emotional change. In short, this configuration can create animacy in the vehicle for the occupant. As a result, the psychological state or physiological state of the occupant can be changed.

### (Second Configuration)

In the second configuration, the vehicle animacy perception promotion system may further include an auditory stimulation application unit outputting a sound containing a voice feature amount based on the action of the occupant applying pressure to the vehicle detected by the occupant pressure detection unit. Thus, this configuration can further promote the occupant's perception of animacy in the vehicle.

### (Third Configuration)

In the first or second configuration, the instruction unit may determine the force to be applied by the actuator to at least one of the two contact units based on the amount of the occupant's action of applying pressure to the vehicle detected by the occupant pressure detection unit, to promote the occupant to perceive animacy in the vehicle. The applied force can provide the occupant with a sense of touch corresponding to the emotional change of the occupant in an enhanced manner. As a result, this configuration can further promote the occupant's perception of animacy in the vehicle.

### (Fourth Configuration)

In any of the first to third configurations, the instruction unit may adjust the force to be applied by the actuator to at least one of the two contact units based on vehicle speed. This configuration changes the sense of touch provided to the occupant in accordance with the state of the vehicle. As a result, the occupant can easily feel the presence of the vehicle as an interactive companion.

### (Fifth Configuration)

A vehicle in the fifth configuration according to another aspect of the present teaching includes: two contact units making contact with different body parts of an occupant of this vehicle; an actuator applying force to at least one of the two contact units; an instruction unit determining the force to be applied by the actuator to at least one of the two contact units based on action detected by an occupant pressure detection unit; and a driving unit driving the actuator. The actuator applies the force to at least one of the two contact units such that the two contact units sandwich a body part of the occupant located between the two contact units to apply pressure to the body part, the force containing a component that brings the two contact units closer to each other in the direction connecting parts of the two contact units contacting the body part. The occupant pressure detection unit detects action of the occupant, who is on the vehicle, applying pressure to the vehicle using his/her muscle strength. The driving unit drives the actuator upon receipt of an instruction indicating the force determined by the instruction unit from the instruction unit. The instruction unit determines the force to be applied by the actuator to at least one of the two contact units based on the action detected by the occupant pressure detection unit in order to promote the occupant to perceive animacy in the vehicle.

The vehicle in the fifth configuration applies pressure to the body parts of the occupant so as to sandwich a body part of the occupant between the two contact units in response to the pressurizing action of the occupant to the vehicle. The pressure can provide the occupant with a sense of touch corresponding to the emotional change of the occupant. Therefore, this configuration can create animacy in the vehicle for the occupant. As a result, the psychological state or physiological state of the occupant can be changed.

### (Sixth Configuration)

In the fifth configuration, the contact units may include a right pressing section making contact with the torso or the right leg of the occupant on the vehicle, and a left pressing section making contact with the torso or the left leg of the occupant, the right pressing section and the left pressing section being aligned in the left-right direction of the vehicle. The actuator may apply force, which brings part of the right pressing section contacting the occupant and part of the left pressing section contacting the occupant closer to each other, to at least one of the right pressing section and the left pressing section. The driving unit can drive the actuator upon receipt of an instruction indicating the force from the instruction unit, the force being determined by the instruction unit based on the action detected by the occupant pressure detection unit, and being applied by the actuator to at least one of the right pressing section and the left pressing section to promote the occupant to perceive animacy in the vehicle. The vehicle animacy perception propulsion system in any of the first to fourth configurations may include the vehicle in the sixth configuration.

### (Seventh Configuration)

In the sixth configuration, the vehicle may include a body frame, a handlebar supported so as to turn at a front portion of the body frame in the front-rear direction of the vehicle, and a seat supported by the body frame and used by the occupant sitting astride. In this configuration, the right pressing section and the left pressing section may be disposed between the handlebar and the seat. The vehicle animacy perception propulsion system in any of the first to fourth configurations may include the vehicle in the seventh configuration.

### (Eighth Configuration)

In the sixth configuration, the vehicle may include the body frame, a handlebar supported so as to turn at a front portion of the body frame in the front-rear direction of the vehicle, and a seat provided further rearward with respect to the handlebar in the front-rear direction of the vehicle and used by the occupant sitting astride. In this configuration, the right pressing section and left pressing section may be located so as to make contact with the occupant sitting astride the seat from the rear in the front-rear direction of the vehicle. The vehicle animacy perception propulsion system in any of the first to fourth configurations may include the vehicle in the eighth configuration.

### (First Program)

The first program according to still another aspect of the present teaching is a vehicle animacy perception promotion program for controlling an actuator. The actuator applies force to at least one of two contact units making contact with different body parts of an occupant on a vehicle such that the two contact units sandwich a body part of the occupant located between the two contact units to apply pressure to the body part, the force containing a component that brings the two contact units closer to each other in the direction connecting parts of the two contact units contacting the body part. The vehicle animacy perception promotion program executed by a computer includes: a process of acquiring a detection result from an occupant pressure detection unit that detects action of the occupant, who is on the vehicle, applying pressure to the vehicle using his/her muscle strength; a process of determining the force to be applied by the actuator to at least one of the two contact units based on the action detected by the occupant pressure detection unit to promote the occupant to perceive animacy in the vehicle; and a process of issuing an instruction indicating the determined force to a driving unit that drives the actuator.

The first program described above can control the actuator so as to sandwich a body part of the occupant between the two contact units to apply pressure to the body part in response to the pressurizing action of the occupant to the vehicle. Therefore, this configuration can create animacy in the vehicle for the occupant. As a result, the psychological state or physiological state of the occupant can be changed. Note that non-transitory recording media storing the vehicle animacy perception promotion program can be included in an embodiment of the present teaching.

### (First Control Method)

The first control method according to still another aspect of the present teaching is a method for controlling an actuator to promote vehicle animacy perception. The actuator applies force to at least one of two contact units making contact with different body parts of an occupant on a vehicle such that the two contact units sandwich a body part of the occupant located between the two contact units to apply pressure to the body part, the force containing a component that brings the two contact units closer to each other in the direction connecting parts of the two contact units contacting the body part. The control method includes: a step of detecting, by using an occupant pressure detection unit, action of the occupant, who is on the vehicle, applying pressure to the vehicle using his/her muscle strength; a step of determining the force to be applied by the actuator to at least one of the two contact units based on the action detected by the occupant pressure detection unit to promote the occupant to perceive animacy in the vehicle; and a step of applying the force to at least one of the contact units by using the actuator that is driven by a driving unit upon receipt of an instruction indicating the determined force. The occupant pressure detection unit may be a sensor. The step of determining the force to be applied by the actuator to at least one of the two contact units may be performed by, for example, a processor.

The first control direction can control the actuator so as to sandwich a body part of the occupant between the two contact units to apply pressure to the body part in response to the pressurizing action of the occupant to the vehicle. Therefore, this configuration can create animacy in the vehicle for the occupant. As a result, the psychological state or physiological state of the occupant can be changed.

The vehicle according to an embodiment of the present teaching includes a body frame, an occupant support unit supported by the body frame and supporting an occupant body, an approach movement detection unit detecting movement of separate body parts of an occupant, supported by the occupant support unit, approaching each other, and a haptic stimulation application unit. The haptic stimulation application unit includes two contact units that are part of the vehicle body and make contact with different body parts of the occupant on the vehicle, an actuator applying force to at least one of the two contact units, the force containing a component that brings the two contact units closer to each other in the direction connecting the two contact units, a controller controlling the force to be applied by the actuator based on the movement detected by the approach movement detection unit (first configuration).

In the first configuration described above, the approach movement detection unit detects movement of the occupant's body parts, which are apart from each other, approaching each other. In other words, the approach movement detection unit detects the approach movement of the occupant's different body parts. For instance, the approach movement includes action performed by the occupant, such as wrapping, grasping, embracing, or sandwiching something. Emotional change of the occupant can be detected by detecting the action. The haptic stimulation application unit applies force to the occupant's body parts in contact with the vehicle by means of the controller, actuator, and contact units based on the movement detected by the approach movement detection unit. The force can provide the occupant with a sense of touch corresponding to the emotional change of the occupant. In addition, the haptic stimulation application unit applies force, which contains a component that brings the two contact units closer to each other in the direction connecting the two contact units in contact with the different body parts of the occupant, to the two contact units. In other words, the haptic stimulation application unit applies force, which contains a component that brings two different body parts, of the whole body part in contact with the vehicle, closer to each other in the direction connecting the two body parts, to at least one of the two contact units. Therefore, the occupant can feel as if his/her two separate body parts are wrapped, embraced, grasped, or sandwiched by the vehicle. Thus, the occupant can feel as if he/she is wrapped or embraced by the vehicle in sync with his/her emotional change. As a result, the degree of attachment of the occupant to the vehicle can be enhanced.

In the first configuration described above, the vehicle may further include an auditory stimulation application unit outputting a sound containing an exclamation voice feature amount based on the action detected by the occupant pressure detection unit (second configuration). This configuration can provide the occupant with a sound corresponding to the emotional change of the occupant together with a sense of touch applied by the haptic stimulation application unit. Therefore, the occupant can feel the vehicle living life. As a result, the degree of attachment of the occupant to the vehicle can be enhanced.

In the first or second configuration described above, the controller of the haptic stimulation application unit can control the actuator to apply the force to at least one of the two contact units in accordance with the amount of the movement detected by the approach movement detection unit (third configuration). The applied force can provide the occupant with a sense of touch corresponding to the emotional change of the occupant in an enhanced manner.

In any of the first to third configurations described above, the controller of the haptic stimulation application unit can adjust the force to be applied by the actuator to at least one of the two contact units based on the vehicle speed (fourth configuration). This configuration changes the sense of touch provided to the occupant in accordance with the state of the vehicle. As a result, the occupant can easily feel the presence of the vehicle as an interactive companion.

In any of the first to fourth configurations described above, the contact units of the haptic stimulation application unit can be configured to include a right pressing section making contact with the torso or the right leg of the occupant supported by the occupant support unit, and a left pressing section making contact with the torso or the left leg of the occupant supported by the occupant support unit, the right pressing section and the left pressing section being supported by the body frame while being aligned in the left-right direction of the vehicle. In this case, the actuator of the haptic stimulation application unit can apply force, which brings the right pressing section and the left pressing section closer to each other, to at least one of the right pressing section and the left pressing section. The controller of the haptic stimulation application unit can control the force to be applied by the actuator to at least one of the right pressing section and the left pressing section in accordance with the movement detected by the approach movement detection unit (fifth configuration). This configuration makes the occupant easily feel as if he/she is wrapped or embraced. Note that the torso in this description includes a waist, abdomen, buttocks, and back. A leg includes from the toe to the base of the leg.

In the fifth configuration described above, the vehicle may further include a handlebar supported so as to turn at a front portion of the body frame. In this case, the occupant support unit includes a seat astride which the occupant sits, and the right pressing section and the left pressing section can be disposed between the handlebar and the seat (sixth configuration). This configuration can achieve a straddled vehicle capable of enhancing the degree of attachment of the occupant to the vehicle.

### BRIEF DESCRIPTION OF DRAWINGS

FIG. 1 is a right-side view of the entire vehicle when viewed from the right of the vehicle.
FIG. 2 is a plan view of the entire vehicle 1 when viewed from above the vehicle.
FIG. 3 is a front view of the entire vehicle 1 when viewed from the front of the vehicle.
FIG. 4 is a right-side view of the entire vehicle with a movable section being in the second position when viewed from the right of the vehicle.
FIG. 5 is a plan view of the entire vehicle with the movable section being in the second position when viewed from above the vehicle.
FIG. 6 is a right-side view of the entire vehicle with the movable section being in the second position when viewed from the front of the vehicle.
FIG. 7 is a block diagram showing a configuration example of a control system built in the vehicle 1.
FIG. 8 is a block diagram showing a modification of the control system.
FIG. 9 is a flowchart showing example process steps for controlling force to be applied to contact units.
FIG. 10 is a block diagram showing a modification of the control system.
FIG. 11 is a flowchart showing example operational steps performed by an auditory stimulation application unit.
FIG. 12 is a left-side view of a vehicle according to a modification.
Fig. 13 illustrates a modification of the contact unit.
FIG. 14 is a left-side view showing a part of the vehicle according to the modification, the part including the contact unit and an actuator.
FIG. 15 is a top view of the part of the vehicle according to the modification, the part including the contact unit and actuator.
FIG. 16 is a perspective view of the part of the vehicle according to the modification, the part including the contact unit and actuator.
FIG. 17 is a cross-sectional view taken along line A-A in FIG. 15. It is a view.
FIG. 18 is a functional block diagram showing a configuration example of a vehicle animacy perception promotion system.
FIG. 19 illustrates a modification of the vehicle animacy perception promotion system.
FIG. 20 illustrates another modification of the vehicle animacy perception promotion system.

### DESCRIPTION OF EMBODIMENT

With reference to the drawings, embodiments of the present teaching will be described in detail below.

In the drawings, Arrow F indicates the forward direction of a vehicle. Arrow B indicates the rearward direction of the vehicle. Arrow U indicates the upward direction of the vehicle. Arrow D indicates the downward direction of the vehicle. Arrow R indicates the rightward direction of the vehicle. Arrow L indicates the leftward direction of the vehicle. Through this description, a front-rear direction of the vehicle, a left-right direction of the vehicle, and an up-down direction of the vehicle denote, respectively, a front-rear direction, left-right direction, and up-down direction with respect to the vehicle as viewed by an occupant operating the vehicle.

A vehicle's body frame occasionally leans in the left direction or in the right direction of the vehicle, or the front direction or the rear direction of the vehicle with respect to the vertical direction. Accordingly, in addition to the directions with respect to the vehicle, directions with respect to the body frame will be defined. In the accompanying drawings, the front-rear direction, left-right direction, and up-down direction of the body frame coincide with the front-rear direction, left-right direction, and up-down direction of the vehicle, respectively. This means that the vehicle in the accompanying drawings is in an upright position without leaning to the left or right.

In this description, a "front-rear direction of the vehicle", a "left-right direction of the vehicle", and an "up-down direction of the vehicle" denote, respectively, a front-rear direction, left-right direction, and up-down direction with respect to the body frame as viewed by an occupant operating the vehicle.

When the following explanation simply cites "forward", "rearward", "leftward", "rightward", "upward", and "downward", these words are defined to denote, respectively, the forward in the front-rear direction of the body frame, the rearward in the front-rear direction of the body frame, the leftward in the left-right direction of the body frame, the rightward in the left-right direction of the body frame, the upward in the up-down direction of the body frame, and the downward in the up-down direction of the body frame.

In this description, the phrase "extending forward in the front-rear direction of the body frame" includes extending in directions inclined with respect to the front direction of the body frame or the rear direction of the body frame. The phrase "extending rearward in the front-rear direction of the body frame" includes extending in directions inclined with respect to the front direction of the body frame or the rear direction of the body frame.

In this description, the phrase "extending leftward in the left-right direction of the body frame" includes extending in directions inclined with respect to the left direction of the body frame or the right direction of the body frame. The phrase "extending rightward in the left-right direction of the body frame" includes extending in directions inclined with respect to the left direction of the body frame or the right direction of the body frame.

In this description, the phrase "extending upward in the up-down direction of the body frame" includes extending in directions inclined with respect to the up direction of the body frame or the down direction of the body frame. The phrase "extending downward in the up-down direction of the body frame" includes extending in directions inclined with respect to the up-down direction of the body frame.

In this description, the phrase "the body frame in an upright position" denotes that the up-down direction of the body frame coincides with the vertical direction. When the body frame is in an upright position, directions with respect to the vehicle coincide with directions with respect to the body frame. When the body frame leans in the left direction or in the right direction with respect to the vertical direction, the left-right direction of the vehicle does not coincide with the left-right direction of the body frame, and the up-down direction of the vehicle also does not coincide with the up-down direction of the body frame. Even when the body frame leans in the left direction or in the right direction with respect to the vertical direction, however, the front-rear direction of the vehicle coincides with the front-rear direction of the body frame.

In this description, the term "connection" includes, in addition to a physical connection, an electrical connection, and establishment of communications. The physical connection includes, for example, a direct connection of two components, and an indirect connection of two components with one or more components therebetween.

### (Configuration of Vehicle)

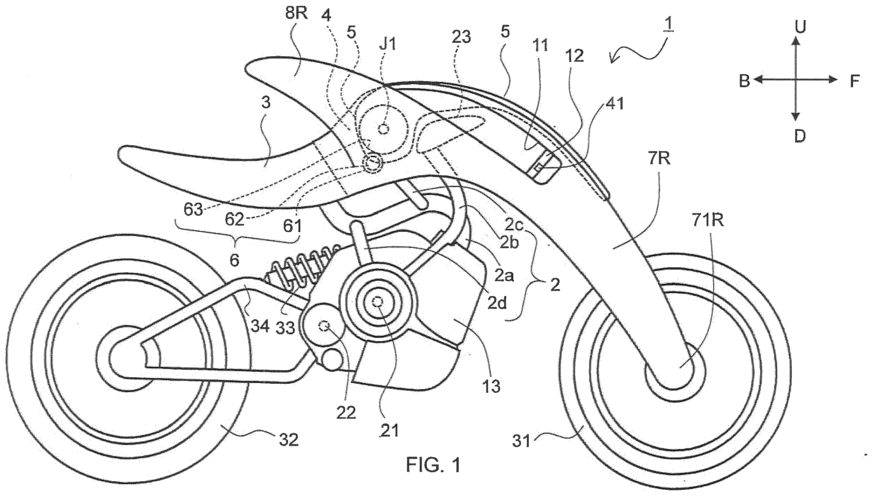

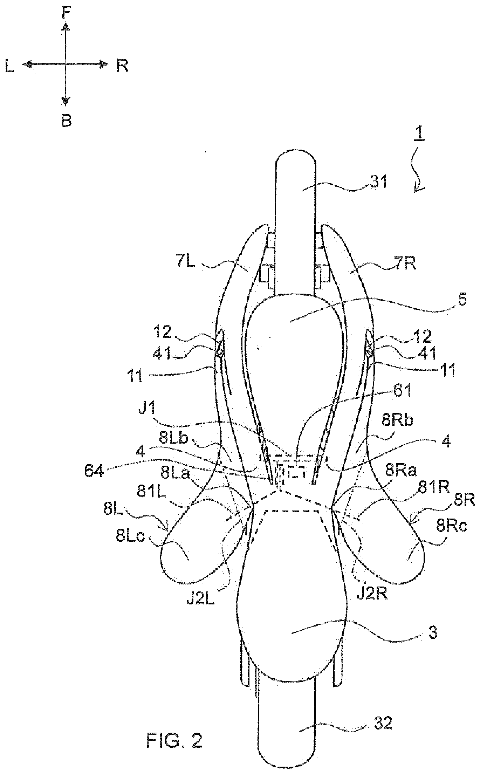

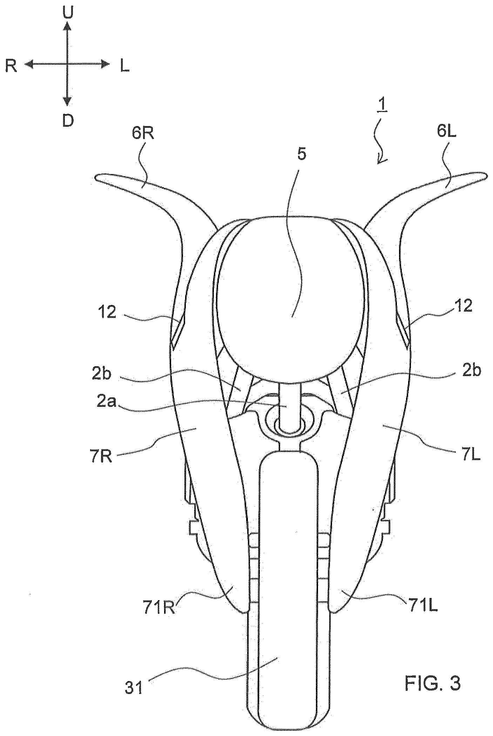

FIG. 1 is a right-side view of the entire vehicle 1 when viewed from the right of the vehicle. FIG. 2 is a plan view of the entire vehicle 1 when viewed from above the vehicle. FIG. 3 is a front view of the entire vehicle 1 when viewed from the front of the vehicle. As shown in FIG. 1, the vehicle 1 includes body frames 2a, 2b, 2c, 2d (hereinafter, the body frames 2a to 2d are collectively referred to as a body frame 2 unless otherwise particularly distinguished from one another), a seat 3, front-wheel support sections 7R, 7L, a front wheel 31, a rear wheel 32, a power unit 13, a rear suspension 33, and a rear arm 34. The front-wheel support sections 7R, 7L are, as shown in FIGS. 2 and 3, a right front-wheel support section 7R and a left front-wheel support section 7L; however, FIG. 1 shows only the right front-wheel support section 7R.

The body frame 2 supports the seat 3, front-wheel support sections 7R, 7L, and power unit 13. The front-wheel support sections 7R, 7L rotatably support the front wheel 31 at the front ends 71R, 71L (FIG. 1 shows only the right front end 71R).

The power unit 13 is suspended by the body frame 2. The power unit 13 includes driving sources, such as an engine, an electric motor, and a battery, and some devices like a transmission.

At a rear part of the body frame 2, the rear arm 34 is pivotally attached about a pivot shaft 22. The rear arm 34 rotatably supports the rear wheel 32 at the rear end. The rear suspension 33 has one end rotatably attached to the rear arm 34, and the other end rotatably attached to the power unit 13. The end of the rear suspension 33 attached to the power unit 13 may be attached to the body frame 2 instead. Although it is not illustrated, the vehicle 1 includes power transmitting members (e.g., chain, belt, and shaft) that transmit driving force generated by the power unit to the rear wheel 32.

In the example shown in FIG. 1, the body frame 2 includes a frame 2a disposed at the center in the left-right direction, and frames 2b, 2c, 2d connected to the frame 2a and disposed on the left and right sides of the frame 2a. The frame 2a is curved and extends in the front-rear direction. An end of the frame 2a is connected to the seat 3. The frame 2a extends forward from the seat 3, is curved downward, and then extends rearward. The other end of the frame 2a, that is the rear end, supports the pivot shaft 22 of the rear arm 34. The frames 2c branch off from the frame 2a, extend upward, and are connected to a base section 4. The frames 2d branch off from the frame 2a behind the branch point of the frames 2c on the frame 2a, extend downward, and then are connected to the frames 2b at a connecting point 21. The frames 2b extend upward from the connecting point 21, pass in front of the frames 2c, and are connected to a guard 23 above the frame 2a. The frames 2b, 2c, 2d are in pairs, each disposed on the left and right sides of the frame 2a. As shown in FIG. 3, the frames 2b are disposed on the left and right sides of the frame 2a.

In addition, the vehicle 1 includes the base section 4 disposed in front of the seat 3, a movable section 5 supported by the base section 4, a right pressing section 8R, and a left pressing section 8L (FIG. 1 shows only the right pressing section 8R). The base section 4 is supported by the body frame 2. The movable section 5 is supported so as to move in the up direction or in the down direction with respect to the base section 4. The right pressing section 8R and left pressing section 8L are supported so as to move in the left-right direction with respect to the base section 4. The vehicle 1 includes actuators 61, 62, 63 (hereinafter, the actuators 61, 62, 63 are collectively referred to as an actuator 6 unless otherwise particularly distinguished from one another) that move the movable section 5, right pressing section 8R, and left pressing section 8L.

The base section 4 is in contact with a front end of the seat 3, and extends forward from the seat 3. As seen from a side (right side) of the vehicle 1, the movable section 5 has a supported portion disposed so as to overlap the base section 4. The movable section 5 is supported by the base section 4 with the supported portion. The supported portion is a pivot shaft J1 of the movable section 5. The axis direction of the pivot shaft J1 coincides with the left-right direction of the body frame 2. The movable section 5 is formed so as to extend forward from the supported portion. The movable section 5 is pivotally supported about the pivot shaft J1. This configuration movably supports the movable section 5 in the up-down direction with respect to the base section 4.

As shown in FIG. 2, the base section 4 is disposed on the left and right sides of the supported portion of the movable section 5. Specifically, the base section 4 extends forward from the seat 3, and branches off leftward and rightward at a midpoint, and the branched parts are connected with the right front-wheel support section 7R and left front-wheel support section 7L, respectively. The pivot shaft J1 of the movable section 5 is disposed between the left and right branched parts of the base section 4. In this example, the base section 4 is formed integrally with the front-wheel support sections 7R, 7L. Specifically, the branched part of the base section 4 disposed on the right side of the movable section 5 is connected with the right front-wheel support section 7R. Similarly, the branched part of the base section 4 disposed on the left side of the movable section 5 is connected with the left front-wheel support section 7L.

As shown in FIG. 2, the base section 4 is formed integrally with the right pressing section 8R and left pressing section 8L. Specifically, the branched part of the base section 4 disposed on the right side of the movable section 5 is connected with the right pressing section 8R. Similarly, the branched part of the base section 4 disposed on the left side of the movable section 5 is connected with the left pressing section 8L. The right pressing section 8R juts out rightward from the base section 4. The left pressing section 8L juts out leftward from the base section 4.

As shown in FIG. 2, the right pressing section 8R is positioned further to the right than the base section 4 when viewed from above. The left pressing section 8L is positioned further to the left than the base section 4 when viewed from above. When viewed from above, the right pressing section 8R has a joint portion 8Ra contiguous to the base section 4, and the left pressing section 8L has a joint portion 8La contiguous to the base section 4, and these joint portions 8Ra, 8La are positioned further to the front than a seating position of an occupant on the seat 3. The rear ends of the right pressing section 8R and left pressing section 8L overlap the seating position of the occupant in the front-rear direction of the body frame 2 as viewed from above. In other words, the rear ends of the right pressing section 8R and left pressing section 8L are positioned further to the rear than the front end of the seat 3.

According to this configuration, the joint portion 8Ra of the right pressing section 8R contiguous to the base section 4 is positioned above the right knee of the occupant sitting astride the seat 3. The joint portion 8La of the left pressing section 8L contiguous to the base section 4 is positioned above the left knee of the occupant sitting astride the seat 3. Because of this, the right pressing section 8R is formed to extend from above the right knee of the occupant sitting astride the seat 3 toward the right side of the occupant's torso. The right pressing section 8R moving to the left pushes the right side of the occupant's torso. The left pressing section 8L moving to the right pushes the left side of the occupant's torso. In short, the right pressing section 8R and left pressing section 8L are configured to support the torso of the occupant. The right pressing section 8R and left pressing section 8L make contact with different parts of the occupant's body, respectively. The right pressing section 8R and left pressing section 8L are an example of contact units making contact with different parts of the occupant. Incidentally, the seat 3 is an example of an occupant support unit that is supported by the body frame to support the occupant's body.

As shown in FIG. 1, the right pressing section 8R partially overlaps a part of the movable section 5 and a part of the base section 4 when viewed from the right. The right pressing section 8R is formed to extend rearward and upward. Although it is not illustrated in FIG. 1, the left pressing section 8L is provided so as to overlap the right pressing section 8R when viewed in the left-right direction of the body frame 2. This means that the left pressing section 8L partially overlaps a part of the movable section 5 and a part of the base section 4 when viewed from the left. The right pressing section 8R and left pressing section 8L are supported by the body frame 2b with the base section 4.

As shown in FIG. 2, in this example, the right pressing section 8R and left pressing section 8L are supported so as to move in the left direction or in the right direction with respect to the base section 4. Specifically, the right pressing section 8R includes a right fixed portion 8Rb and a right jutting movable portion 8Rc. The right fixed portion 8Rb is supported by the body frame 2. The right jutting movable portion 8Rc is movably supported by the right fixed portion 8Rb, and extends rightward and rearward from the right fixed portion 8Rb. The right jutting movable portion 8Rc is supported so as to turn about a rotational axis J2R, which is a line along a right edge of the right fixed portion 8Rb. Turning the right jutting movable portion 8Rc about the rotational axis J2R changes the position of the right jutting movable portion 8Rc in the left direction or in the right direction. In this example, the rotational axis J2R is inclined with respect to the axis of the body frame 2 in the front direction of the body frame 2 or in the rear direction of the body frame 2. The front end of the rotational axis J2R is positioned further to the right than the rear end thereof.

The left pressing section 8L includes a left fixed portion 8Lb and a left jutting movable portion 8Lc. The left fixed portion 8Lb is supported by the body frame 2. The left jutting movable portion 8Lc is movably supported by the left fixed portion 8Lb, and extends leftward and rearward from the left fixed portion 8Lb. The left jutting movable portion 8Lc is supported so as to turn about a rotational axis J2L, which is a line along a left edge of the left fixed portion 8Lb. Turning the left jutting movable portion 8Lc about the rotational axis J2L changes the position of the left jutting movable portion 8Lc in the left direction or in the right direction. In this example, the rotational axis J2L is inclined with respect to the axis of the body frame 2 in the front direction of the body frame 2 or in the rear direction of the body frame 2. The front end of the rotational axis J2L is positioned further to the left than the rear end thereof.

The actuator 6 for moving the right pressing section 8R and left pressing section 8L can use, for example, a motor 61 as a power source. In the example shown in FIG. 2, wires 81R, 81L are provided as a means to transmit driving force of the motor 61. The wires 81R, 81L are taken up by a take-up device 64 secured to the body frame 2. The take-up device 64 is driven by the motor 61. One end of the wire 81R is attached to the take-up device 64. The other end of the wire 81R is attached to the right jutting movable portion 8Rc away from the rotational axis J2R. The wire 81R intersects with the rotational axis J2R. One end of the wire 81L is attached to the take-up device 64. The other end of the wire 81L is attached to the left jutting movable portion 8Lc away from the rotational axis J2L. The wire 81L intersects with the rotational axis J2L. When the take-up device 64 takes up the wires 81R, 81L, the wire 81R pulls the right jutting movable portion 8Rc thereby to turn the right jutting movable portion 8Rc about the rotational axis J2R, while the wire 81L pulls the left jutting movable portion 8Lc thereby to turn the left jutting movable portion 8Lc about the rotational axis J2L.

The turn of the right jutting movable portion 8Rc of the right pressing section 8R about the rotational axis J2R moves the right jutting movable portion 8Rc in the left direction or in the right direction with respect to the base section 4. The turn of the left jutting movable portion 8Lc of the left pressing section 8L about the rotational axis J2L moves the left jutting movable portion 8Lc in the left direction or in the right direction with respect to the base section 4. The wires 81R, 81L taken up concurrently move the right jutting movable portion 8Rc leftward, and the left jutting movable portion 8Lc rightward, respectively. In other words, the right jutting movable portion 8Rc and left jutting movable portion 8Lc move as if they approach each other. This movement allows the right pressing section 8R and left pressing section 8L to press the occupant's torso from the left and right, respectively. Specifically, the movement generates force containing a component bringing the two contact units closer to each other in the direction connecting the two contact units that make contact with different parts of the occupant's body (a part of the right pressing section 8R contacting the body and a part of the left pressing section 8L contacting the body).

The mechanism to move the right pressing section 8R and left pressing section 8L closer to each other is not limited to the aforementioned example. For instance, both the right pressing section 8R and left pressing section 8L can be provided with a motor. Specifically, a right motor for driving the right pressing section 8R and a left motor for driving the left pressing section 8L can be installed. In this case, for example, the right motor can be disposed so that its output shaft is arranged in parallel with the rotational axis J2R of the right jutting movable portion 8Rc. In addition, a transferring member may be provided to transfer the rotation of the output shaft of the right motor to the right jutting movable portion 8Rc. This configuration allows the right jutting movable portion 8Rc to turn about the rotational axis J2R with the rotation of the output shaft of the right motor. Similarly, for example, the left motor can be disposed so that its output shaft is arranged in parallel with the rotational axis J2L of the left jutting movable portion 8Lc. In addition, a transferring member may be provided to transfer the rotation of the output shaft of the left motor to the left jutting movable portion 8Lc. This configuration allows the left jutting movable portion 8Lc to turn about the rotational axis J2L with the rotation of the output shaft of the left motor. The right motor and left motor can be controlled with, for example, the same control signal.

In another example, each of the right pressing section 8R and left pressing section 8L may be configured to include, for example, an airbag at the part abutting against the occupant. In this case, a compressor serving as an actuator, and an air pipe connecting the compressor and airbag can be provided to the vehicle 1. The compressor can be driven by, for example, a motor or an engine. The air pipe can be provided with an air valve. The amount of air filled in the airbags can be adjusted with the pressure from the compressor by controlling the closing and opening of the air valve. Increasing the amount of air in the airbags of the right pressing section 8R and left pressing section 8L moves the airbags of the right pressing section 8R and left pressing section 8L closer to each other. This movement can apply force on the occupant in a direction in which different body parts of the occupant approach each other.

The actuator may be configured to include both the motor and the compressor for the airbags. For instance, the vehicle 1 can be equipped with both a mechanism for turning the right jutting movable portion 8Rc and left jutting movable portion 8Lc about the rotational axes J2R and J2L, respectively, by using the motor 61, and a mechanism for inflating the right jutting movable portion 8Rc and left jutting movable portion 8Lc by using the airbags.

In the foregoing example, the actuator 6 that moves the right pressing section 8R and left pressing section 8L includes the motor 61, take-up device 64, and wires 81R, 81L. On the contrary, the actuator 6 that moves the movable section 5 includes, as shown in FIG. 1, the motor 61, and speed reducers (gears) 62, 63. The rotation of the output shaft of the motor 61 is transferred to the pivot shaft J1 via the speed reducers 62, 63. In this example, the output shaft of the motor 61 and the rotational shafts of the speed reducers 62, 63 are in parallel with the pivot shaft J1. The speed reducer 62 is coaxial with the output shaft of the motor 61, and rotates with the rotation of the output shaft of the motor 61. The speed reducer 63 is coaxial with the pivot shaft J1 and meshes with the speed reducer 62. With the rotation of the speed reducer 62, the speed reducer 63 rotates. With the rotation of the speed reducer 63, the pivot shaft J1 rotates. The configuration of the actuator 6 to move the movable section 5 is not limited to the example shown in FIG. 1.

As shown in FIG. 1, the front-wheel support section 7R extends forward and downward from the base section 4 toward the front wheel 31. The front-wheel support section 7R is continuously formed with the base section 4. Although it is not illustrated in FIG. 1, the front-wheel support section 7L extends forward and downward from the base section 4 toward the front wheel 31 through the left side of the movable section 5 (see FIGS. 2 and 3). The front-wheel support section 7L is continuously formed with the base section 4.

As shown in FIG. 1, a groove 11 is formed on a right side face of the front-wheel support section 7R so as to extend forward and downward from the base section 4 toward the front wheel 31. The groove 11 is a recessed portion in the right side face of the front-wheel support section 7R, and is recessed toward the inside of the vehicle. The groove 11 has a bottom face extending forward and downward, and a pair of side faces opposed to each other in a direction perpendicular to the direction in which the bottom face extends. Inside the recessed portion formed in the groove 11, a grip section 12 is provided. The grip section 12 is a rod-like member extending from one of the paired opposed side faces of the groove 11 to the other side face.

Although it is not illustrated in FIG. 1, a groove 11 is formed on a left side face of the front-wheel support section 7L so as to extend forward and downward from the base section 4 toward the front wheel 31 (See FIGS. 2 and 3). The groove 11 is a recessed portion of the left side face of the front-wheel support section 7L, and is recessed toward the inside of the vehicle. The groove 11 includes a bottom face extending forward and downward, and a pair of side faces opposed to each other in a direction perpendicular to the direction in which the bottom face extends. Inside the recessed portion formed in the groove 11, a grip section 12 is provided. The grip section 12 is a rod-like member extending from one of the paired opposed side faces of the groove 11 to the other side face.

The grip section 12 is a part that an occupant sitting astride the seat 3 holds. The grip section 12 is provided with a pressure sensor 41. The pressure sensor 41 detects pressure applied on the grip section 12 by the hands of the occupant. The pressure sensor 41 can detect action of the occupant holding the grip section 12, that is, movement of the occupant's fingers and palm approaching each other. The pressure sensor 41 is an example of an approach movement detection unit that detects that the occupant's body parts, which are apart from each other, are approaching each other. The occupant's fingers and palm correspond to the occupant's body parts apart from each other. The grip section 12 may be provided with a brake operation element.

The upper face of the base section 4 is continuously formed with the upper faces of the seat 3, front-wheel support sections 7L, 7R, right pressing section 8R, and left pressing section 8L. For instance, a cover placed over the front-wheel support sections 7L, 7R, base section 4, seat 3, right pressing section 8R, and left pressing section 8L can make their upper faces one continuous configuration.

### (Operation Example)

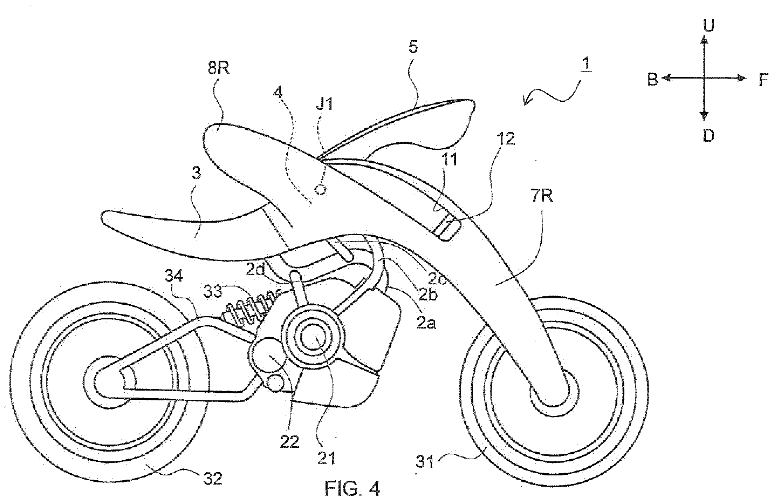

FIGS. 1, 2, and 3 show the vehicle 1 with the right pressing section 8R and left pressing section 8L being in the first position. FIGS. 4, 5, and 6, on the contrary, show the vehicle 1 with the right pressing section 8R and left pressing section 8L being in the second position.

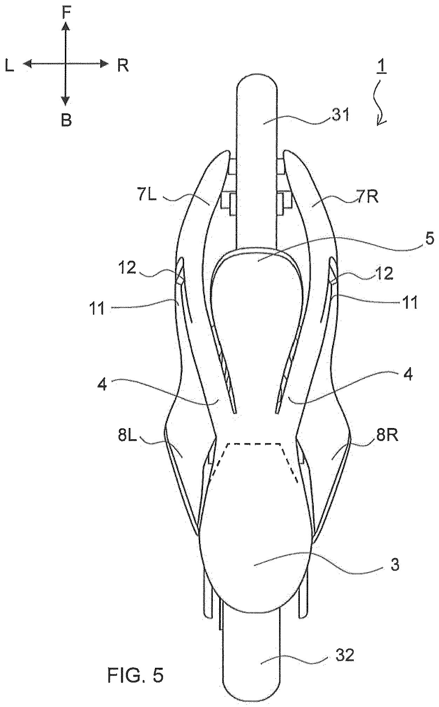

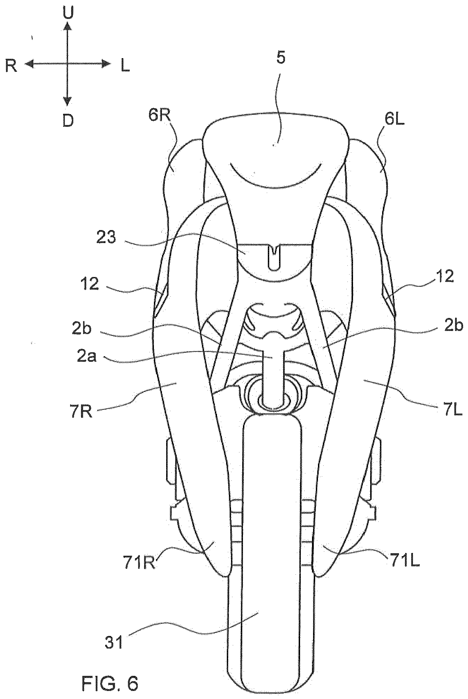

As shown in FIGS. 2 and 3, the right pressing section 8R in the first position has the right jutting movable portion 8Rc turned about the rotational axis J2R in a direction in which it is inclined rightward with respect to the up-down direction line of the body frame 2, while the left pressing section 8L in the first position has the left jutting movable portion 8Lc turned about the rotational axis J2L in a direction in which it is inclined leftward with respect to the up-down direction line of the body frame 2. On the other hand, as shown in FIGS. 5 and 6, the right pressing section 8R in the second position has the right jutting movable portion 8Rc turned about the rotational axis J2R in a direction it approaches the up-down direction line of the body frame 2, while the left pressing section 8L in the second position has the left jutting movable portion 8Lc turned about the rotational axis J2L in a direction in which it approaches the up-down direction line of the body frame 2.

The distance between a right edge of the right pressing section 8R and a left edge of the left pressing section 8L in the left-right direction when the right pressing section 8R and left pressing section 8L are in the second position (see FIG. 5) is shorter than the distance between the right edge of the right pressing section 8R and the left edge of the left pressing section 8L in the left-right direction when the right pressing section 8R and left pressing section 8L are in the first position (see FIG. 2). In short, the space between right pressing section 8R and left pressing section 8L in the second position is narrower than that in the first position. In other words, the right pressing section 8R and left pressing section 8L in the second position are closer to each other than they are in the first position.

As shown in FIG. 6, when the right pressing section 8R and left pressing section 8L are in the second position, the right pressing section 8R and left pressing section 8L are not inclined in the left direction or in the right direction with respect to the up-down direction line of the body frame 2. As shown in FIG. 3, when the right pressing section 8R and left pressing section 8L are in the first position, the right pressing section 8R and left pressing section 8L are inclined in the left direction or in the right direction with respect to the up-down direction line of the body frame 2. This means that the actuator can incline the right pressing section 8R rightward with respect to the up-down direction axis of the body frame 2. This also means that the actuator can incline the left pressing section 8L leftward with respect to the up-down direction axis of the body frame 2.

The actuator 6 can move the right pressing section 8R and left pressing section 8L between the first position and the second position. The actuator 6 applies force containing a component bringing the right pressing section 8R and left pressing section 8L closer to each other. The force applied by the actuator 6 is controlled based on the occupant's grip action detected by the pressure sensor 41.

### (Configuration Example of Control System)

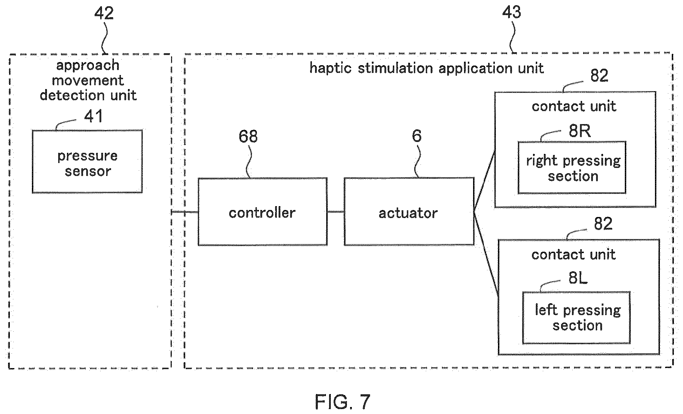

FIG. 7 is a block diagram showing a configuration example of a control system in the vehicle 1. The control system shown in FIG. 7 includes an approach movement detection unit 42 and a haptic stimulation application unit 43. The approach movement detection unit 42 detects movement in which body parts, which are apart from each other, of an occupant supported by a seat 3 (occupant support unit) are approaching each other. The approach movement detection unit 42 includes, as an example, a pressure sensor 41. The pressure sensor 41 detects pressure applied on the grip section 12 by the hands of the occupant. The haptic stimulation application unit 43 includes a controller 68, an actuator 6, and two contact units 82. The two contact units 82 are part of the vehicle, and make contact with different body parts of the occupant. In an example, the two contact units 82 include the right pressing section 8R and left pressing section 8L, respectively. The actuator 6 applies force, which contains a component that brings the two contact units 82 closer to each other in the direction connecting the two contact units 82, to at least one of the two contact units 82. In an example, the actuator 6 applies force, which contains a component bringing the right pressing section 8R and left pressing section 8L closer to each other, to the right pressing section 8R and left pressing section 8L. The controller 68 controls the force to be applied by the actuator 6 to the contact units 82 based on the occupant's movement detected by the approach movement detection unit 42.

Information about the occupant's movement detected by the approach movement detection unit 42 is transmitted to the controller 68. In short, the controller 68 acquires detection results of the approach movement detection unit 42. In this example, the controller 68 receives a signal indicating the pressure on the grip section 12 detected by the pressure sensor 41. The controller 68 generates a control signal based on the received signal indicating the pressure, and feeds the generated control signal to the actuator 6. In an example, the controller 68 can control the actuator 6 by feeding a signal indicating a current value for the motor 61 of the actuator 6, to the motor 61.

The controller 68 can be made up with a computer, such as an ECU, or electronic circuitry. The computer or electronic circuitry making up the controller 68 is mounted on a substrate that may be built in or out of a case where the actuator 6 is housed.

The controller 68 controls the actuator 6 to apply force, which contains a component that brings two parts of the contact units 82 closer to each other, to the contact units 82 when the occupant's movement detected by the approach movement detection unit 42 satisfies predetermined conditions. In an example, when the pressure detected by the pressure sensor 41 exceeds a threshold, the controller 68 controls the actuator 6 to move the right pressing section 8R and left pressing section 8L closer to each other. This configuration enables the control of the right pressing section 8R and left pressing section 8L to press the occupant's torso from the left and right, respectively, when the gripping force generated by the occupant holding the grip section 12 exceeds a predetermined magnitude.

In addition, the controller 68 may adjust the magnitude of the force to be applied by the actuator 6 to the contact units 82, or the timing at which the force is applied by the actuator 6 to the contact units 82 in accordance with the occupant's movement detected by the approach movement detection unit 42. For instance, the controller 68 can change the magnitude of the force to be applied to the contact units 82 in accordance with the amount of the detected occupant's movement. In a specific example, the controller 68 can control the magnitude of the force with which the actuator 6 moves the right pressing section 8R and left pressing section 8L in accordance with the pressure detected by the pressure sensor 41. This can change the pressing force applied by the right pressing section 8R and left pressing section 8L to the occupant's torso from the left and right, respectively, in accordance with, for example, the gripping force applied by the occupant.

In addition to the magnitude of the force to be applied by the actuator 6 to the contact units 82, the controller 68 may control the speed to move the contact units 82, or the timing at which the actuator 6 applies the force, in accordance with the occupant's movement detected by the approach movement detection unit.

Thus, the controller 68 can determine a control value (control data) to control the force applied by the actuator 6 from a detection value that indicates the occupant's movement detected by the approach movement detection unit 42. The process to determine the control value for the actuator 6 using a value indicating the occupant's movement can include, for example, a process of calculating the control value using predetermined mathematical expressions with the detection value as a parameter. Alternatively, the controller 68 can use data (e.g., table or map data) containing various detection values and control values associated therewith to perform the process to determine a control value in association with the detection acquired from the approach movement detection unit 42.

As the occupant's movement detected by the approach movement detection unit 42, the controller 68 may receive information from two or more sensors. For instance, in addition to the pressure sensor 41 of the grip section 12, a knee-grip pressure sensor for detecting gripping pressure of the occupant's knees can be provided to the vehicle 1. In this case, the controller 68 can control the force to be applied by the actuator 6 to the right pressing section 8R and left pressing section 8L using signals acquired from both the pressure sensor of the grip section 12 and the knee-grip pressure sensor.

The actuator 6 may be configured to apply force to two or more pairs of the contact units 82. For instance, in addition to the right pressing section 8R and left pressing section 8L, another pair of contact units can be provided to the vehicle 1. An example of the contact units can be airbags disposed in the grooves 11 so as to surround the grip section 12. The airbags can be configured to press the fingers and the back of the occupant's hands holding the grip section 12 by inflating the airbags. In this case, a compressor serving as the actuator 6, an air pipe connecting the compressor and airbag, and an air valve provided to the air pipe can be provided to the vehicle 1. The controller 68 controls both the actuator 6 that moves the right pressing section 8R and left pressing section 8L, and the air valve. The controller 68 can perform the control operations based on the occupant's movement detected by the approach movement detection unit 42.

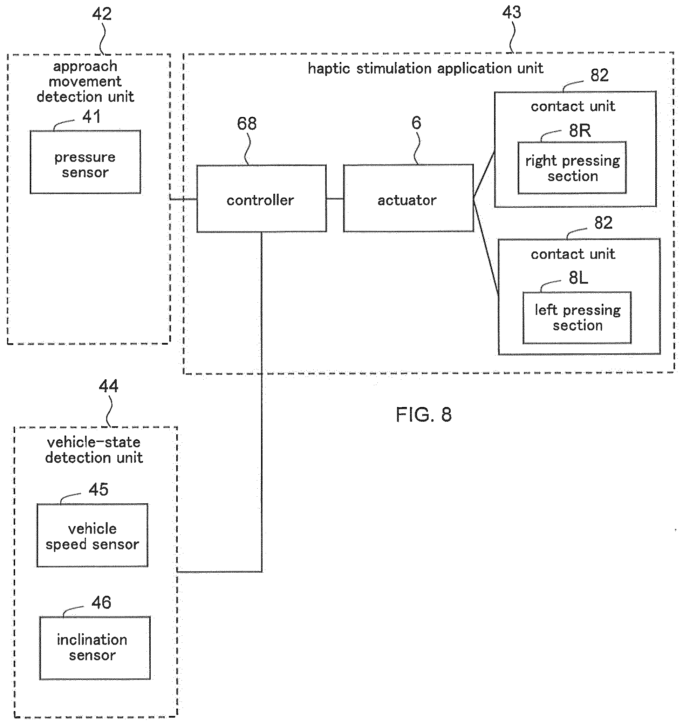

In addition, the controller 68 can control the force to be applied by the actuator 6 to the contact units 82 based on information about the state of the vehicle, in addition to the occupant's movement detected by the approach movement detection unit 42. FIG. 8 is a block diagram showing a configuration example of a control system capable of controlling the force to be applied by the actuator to the contact units 82 using the state of the vehicle. The control system shown in FIG. 8 includes a vehicle-state detection unit 44. The vehicle-state detection unit 44 is provided in the vehicle 1, and detects a physical quantity indicating the state of the vehicle. In an example, the vehicle-state detection unit 44 includes a vehicle speed sensor 45 and an inclination sensor 46. The controller 68 controls the force to be applied by the actuator 6 to the contact units 82 based on the vehicle's state detected by the vehicle-state detection unit 44. For instance, the controller 68 can adjust the force to be applied by the actuator 6 to the contact units 82 in accordance with the speed of the vehicle detected by the vehicle speed sensor 45.

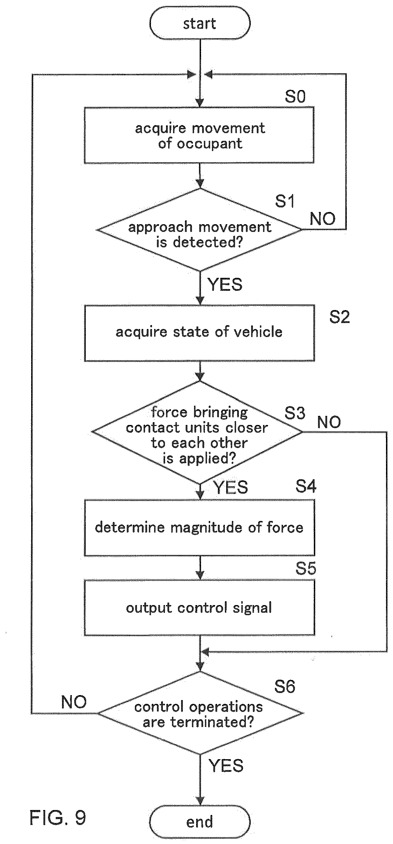

FIG. 9 is a flowchart showing an example of a process to control the force to be applied to the contact units based on the occupant's movement and vehicle's state. In the example shown in FIG. 9, the controller 68 acquires information about an occupant's movement from the approach movement detection unit 42 (S0). For instance, the controller 68 acquires a signal, which indicates the pressure exerted on the grip section 12 by the driver's hands, from the pressure sensor 41 provided on the grip section 12.

The controller 68 detects by means of the signal from the approach movement detection unit 42 that the occupant has moved his/her separate body parts closer to each other (hereinafter, this movement is referred to as approach movement) (YES in S1). For instance, the controller 68 determines that there is an approach movement when the pressure detected by the pressure sensor 41 on the grip section 12 exceeds a threshold. In this example, the pressure sensor 41 detects movement of the fingers and palm, which are regarded as separate body parts of the occupant, approaching each other.

Upon detection of the approach movement, the controller 68 acquires information indicating the state of the vehicle from the vehicle-state detection unit 44 (S2). For instance, as the information indicating the vehicle's state, the controller 68 acquires a signal indicating a physical quantity that indicates the state of the vehicle derived from the occupant's movement. In a more specific example, the controller 68 acquires a signal indicating the speed of the vehicle from the vehicle speed sensor 45.

The controller 68 determines whether or not to apply force that brings the contact units 82 closer to each other based on the vehicle's state acquired in S2 (S3). For instance, the controller 68 determines application of force when the vehicle's speed detected by the vehicle speed sensor 45 is in a predetermined range (YES in S3). If it is determined not to apply force (NO in S3), the controller 68 performs a process to determine whether or not to terminate the control operations (S6).

In the case of YES in S3, the controller 68 determines the magnitude of the force that brings the contact units 82 closer to each other (S4). In S4, the controller 68 can determine the magnitude of the force using the approach movement detected in S1 and the information about the vehicle's state acquired in S2. For instance, the magnitude of the force that brings the contact units 82, namely, the right pressing section 8R and left pressing section 8L, closer to each other is determined in accordance with the pressure acquired by the pressure sensor 41 in S1 and the speed of the vehicle acquired in S2. The determination of the magnitude of the force associated with the pressure acquired by the pressure sensor 41 in S0 and the speed of the vehicle acquired in S1 can be made, for example, by referring to correlation data between previously-stored vehicle's states and the magnitudes of forces to be applied. Incidentally, in S4, the controller 68 may determine other control items, such as the timing to apply the force or the speed to move the contact units 82, instead of, or in addition to the magnitude of the force.

The controller 68 feeds a control signal based on the item determined in S4 to the actuator 6 (S5). With this signal, the actuator 6 applies force with a magnitude determined in S4 to the contact units 82. The controller 68 repeats the process steps from S0 to S5 until it determines to terminate the control operations (YES in S6).

The operations of the controller 68 are not limited to the example shown in FIG. 9. For instance, the controller 68 may omit the process step S3 in FIG. 9. The process step S2 can be also omitted. In this case, in S4, the force to be applied is determined based on the approach movement of the occupant acquired in S0. In addition, the vehicle's state acquired in S2 is not limited to the speed of the vehicle, and can be any other states of the vehicle, such as an amount of brake operation, a throttle opening degree, an amount of leaning or leaning speed in the left direction or in the right direction, a slip ratio, or an engine RPM, instead of or, in addition to, the speed of the vehicle. The process to determine the output voice in S4 is not limited to the aforementioned process using the previously-stored correlation data. The magnitude or speed of the force or the timing to apply the force can be determined by, for example, assigning a value indicating the occupant's movement or vehicle's state into a predetermined mathematical expression or algorithm.

Through the operations shown in FIG. 9, the gripping pressure exerted on the grip section 12 by the occupant can be returned to the torso of the occupant via the contact units 82. The vehicle 1 can provide a haptic stimulus to the occupant in accordance with the gripping action of the occupant's hands. Because the occupant's torso is compressed when the occupant holds the hands of a person, the occupant feels as if he/she is being held by the vehicle 1.

### (Configuration Example including Auditory Stimulation Application Unit)

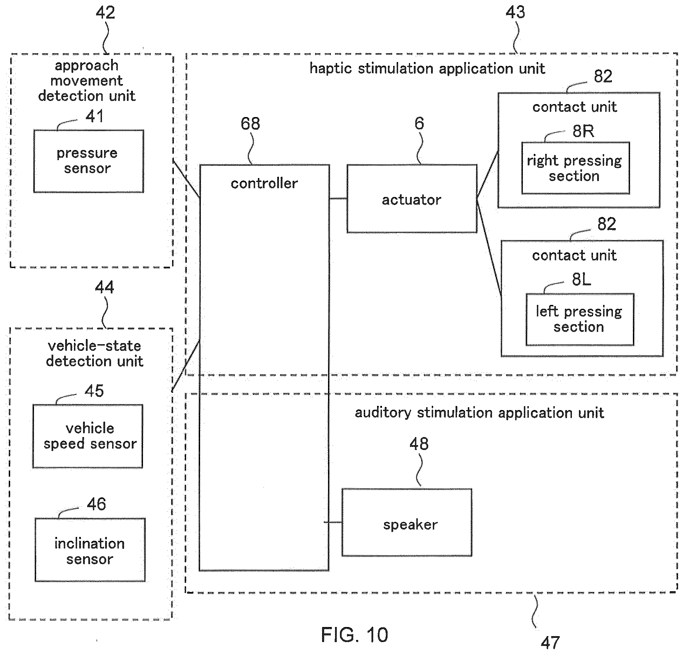

FIG. 10 is a block diagram showing a modification of the control system provided in the vehicle 1. The control system shown in FIG. 10 includes an auditory stimulation application unit 47 in addition to the approach movement detection unit 42 and haptic stimulation application unit 43. The auditory stimulation application unit 47 outputs a sound containing an exclamation voice feature amount based on the movement detected by the approach movement detection unit 42. The auditory stimulation application unit 47 includes a controller 58 and a speaker 48.

The controller 58 of the auditory stimulation application unit 47 can determine whether or not to output (whether or not to require to output) an exclamation sound based on the movement detected by the approach movement detection unit 42. Specifically, the controller 58 determines whether or not to output an exclamation sound based on the movement of body parts, which are apart from each other, of an occupant sitting astride a seat 3 approaching each other, detected by the approach movement detection unit 42. For instance, the controller 58 can determine output of an exclamation sound when the amount of the detected movement is in a predetermined range. In this case, the controller 58 controls the speaker 48 to output the exclamation sound.

Alternatively, the controller 58 of the auditory stimulation application unit 47 may determine the exclamation sound to be output based on the movement detected by the approach movement detection unit 42. For instance, the controller 58 can select an audio data item to be output from previously-stored audio data items of exclamation sounds based on the movement detected by the approach movement detection unit 42. The controller 58 controls the speaker 48 to output the selected audio data item of an exclamation sound. For instance, the controller 58 can change the exclamation sound to be output in accordance with the amount of the detected movement.

The auditory stimulation application unit 47 that outputs an exclamation sound based on the movement detected by the approach movement detection unit 42 can therefore output the exclamation sound in sync with application of force by the haptic stimulation application unit 43 via the contact units 82. The controller 58 can control the haptic stimulation application unit 43 to output an exclamation sound concurrently with the application of force by the contact units 82 to the occupant's body. In short, application of force by the haptic stimulation application unit 43 can be brought into sync with output of an exclamation sound by the auditory stimulation application unit 47. For instance, the haptic stimulation application unit 43 can apply force at the same time when the auditory stimulation application unit 47 outputs an exclamation sound. Therefore, the occupant can feel a stronger attachment to the vehicle 1.

The exclamation sounds are voices expressing human emotions, and they are not limited to language. The exclamation sounds express human emotions by changing, for example, the pitch or volume of breath sounds. In addition, the exclamation sounds may be syntheses of the breath sounds uttered by humans and other types of sounds (e.g., engine sound, etc.). Examples of the exclamation sounds include a scream, a cry, a laugh, a gasp, a sigh, or a whistle. The scream and cry express surprise, anger, grief, and so on. The laugh and whistle express relaxation, amazement, happiness, and so on. The gasp expresses excitement, pain, pleasure, and so on. The sigh expresses tiredness, despair, and so on. Output of the exclamation sounds can express various emotions without the help of language.

For instance, when the occupant holds the grip section 12 tightly, the haptic stimulation application unit 43 and auditory stimulation application unit 47 can increase the pressure applied by the contact units 82 to hold the torso of the occupant, and also can present a synthesized sound of an exclamation sound and an engine sound. This offers the occupant a stronger sense of being held by the vehicle 1. Therefore, the occupant can feel a stronger attachment to the vehicle 1.

The auditory stimulation application unit 47 may be configured to output an exclamation sound when application of force to the occupant's body from the contact units 82 is determined at the haptic stimulation application unit 43. In this case, the auditory stimulation application unit 47 is configured to output an exclamation sound based on the indirectly-obtained detection result of the approach movement detection unit 42. Such configuration is also regarded as output of an exclamation sound based on the movement detected by the approach movement detection unit 42.

The controller 58 of the haptic stimulation application unit 43 may control the output of an exclamation sound using the state of the vehicle in addition to the movement detected by the approach movement detection unit 42. For instance, when the controller 58 determines output of an exclamation sound based on the movement detected by the approach movement detection unit 42, the controller 58 can determine the exclamation sound to be output based on the vehicle's state detected by the vehicle-state detection unit 44. This enables output of an appropriate exclamation sound in accordance with the vehicle's state.

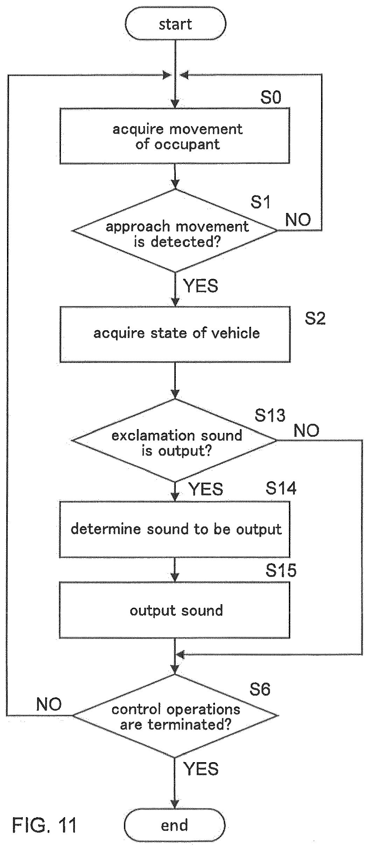

FIG. 11 is a flowchart of example process steps performed by the auditory stimulation application unit 47. S0, S1, and S2 in FIG. 11 can be the same process steps as S0, S1, and S2 in FIG. 9.

The controller 68 of the auditory stimulation application unit 47 determines whether or not to output an exclamation sound based on the vehicle's state acquired in S2 (S13). For instance, the controller 68 determines the output of an exclamation sound when the vehicle speed detected by the vehicle speed sensor 45 is in a predetermined range (YES in S13). If it is determined not to apply force (NO in S13), the controller 68 performs a process to determine whether or not to terminate the control operations (S6).

In the case of YES in S13, the controller 68 determines the exclamation sound to be output (S14). In S14, the controller 68 can determine the exclamation sound to be output using information about the approach movement detected in S1 and the vehicle's state acquired in S2. For instance, the exclamation sound is determined in accordance with the pressure acquired by the pressure sensor 41 in S0 and the vehicle speed acquired in S2.

In one example, the controller 58 can store audio data files containing a plurality of exclamation sounds into an accessible memory in advance. In addition, condition data items containing conditions for detecting approach movements and a vehicle's states are stored in the memory in advance in association with the audio data files. The controller 58 refers to the condition data items to identify a condition data item that satisfies the conditions of the approach movement acquired in S0 and vehicle's state acquired in S2. The audio data file associated with the identified condition data item is determined to be an exclamation sound to be output.

The controller 68 generates an audio signal by reproducing the audio data file determined in S14, and feeds the audio signal to the speaker 48 (S15). Thus, the speaker 48 outputs the exclamation sound determined in S14. The controller 68 repeats the process steps from S0 to S15 until it determines to terminate the control operations (YES in S6).

Through the operations shown in FIG. 11, the gripping pressure exerted on the grip section 12 by the occupant can be returned to the torso of the occupant via the contact units 82. The vehicle 1 can provide a haptic stimulus to the occupant in accordance with the gripping action of the occupant's hands. Because the occupant's torso is compressed when the occupant holds the hands of a person, the occupant feels as if he/she is being held by the vehicle 1.

The operations of the auditory stimulation application unit 47 are not limited to the example shown in FIG. 11. For instance, the process step S13 in FIG. 11 may be omitted. The process step S2 can be also omitted. In this case, in S14, the exclamation sound is determined based on the approach movement of the occupant acquired in S0. In addition, the vehicle's state acquired in S2 is not limited to the speed of the vehicle, and can be any other states of the vehicle, such as an amount of brake operation, a throttle opening degree, an amount of leaning or leaning speed in the left direction or in the right direction, a slip ratio, or an engine RPM, instead of, or in addition to, the speed of the vehicle. The process to determine the output sound in S14 is not limited to the aforementioned process using the previously-stored condition data. The exclamation sound can be determined by, for example, assigning a value indicating the occupant's movement or vehicle's state into a predetermined mathematical expression or algorithm.

The embodiment of the present teaching has been described above; however, the present teaching is not limited to the above-described embodiment.

### (Modification of Vehicle)

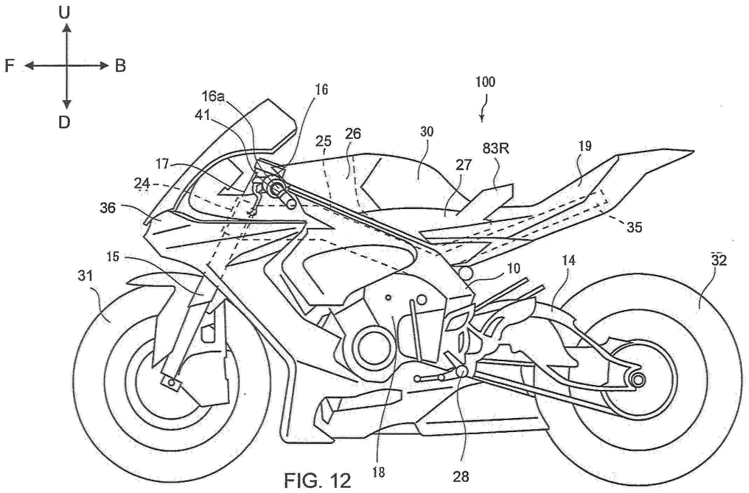

For instance, the vehicle can be equipped with a head pipe and a handlebar at a front part of the body frame. FIG. 12 is a left side view of the vehicle according to a modification. The vehicle 100 shown in FIG. 12 is a motorcycle. The vehicle 100 includes a body frame 10, a power unit 18, a front wheel 31, a rear wheel 32, and a fuel tank 30.

The body frame 10 includes a head pipe 24, a tank frame 25, a rear frame 35, and a rear arm 14. The head pipe 24 is located at a front part of the body frame 10. The tank frame 25 is composed of a pair of a left part and a right part. The tank frame 25 extends rearward from the head pipe 24. When the vehicle 100 is viewed from a side, the left and right parts of the tank frame 25 have downwardly curved portions, respectively. The rear frame 35 composed of a pair of a left part and a right part is connected to the tank frame 25 at the curved portions. The rear arm 14 is connected to a rear end of the tank frame 25. At the rear ends of the left part and right part of the tank frame 25, steps 28 are attached, respectively, so as to project in the left-right direction.

A steering shaft 17 is inserted in the head pipe 24 so as to turn in the head pipe 24. At an upper part of the steering shaft 17 attached is a handlebar 16. A front fork 15 composed of a pair of a left part and a right part is attached to the steering shaft 17 with a bracket (not shown). The front fork 15 rotatably supports a front wheel 31 at the lower end. The front wheel 31 turns in the left direction or in the right direction by manipulating the handlebar 16. The rear arm 14 rotatably supports a rear wheel 32 at the lower end. The rear wheel 32 receives power from the power unit 18 to rotate.

The fuel tank 30 is attached to the tank frame 25 and rear frame 35. As viewed from a side of the vehicle 100, a seat 19 is disposed at the rear of the fuel tank 30. A front part of the seat 19 partially covers a rear part of the fuel tank 30.

The handlebar 16 includes grips 16a that the occupant holds. A pressure sensor 41 is attached to the grips 16a of the handlebar 16. The pressure sensor 41 detects movement of the occupant holding the grip 16a, that is, movement of the occupant's fingers and palm approaching each other.

In front of the handlebar 16, a front cowl 36 is disposed. A cover 26 is disposed at the rear of the handlebar 16. A rear part of the cover 26 partially covers a front part of the fuel tank 30. A base section 27 composed of a pair of a left part and a right part is disposed below the cover 26. The base section 27 is attached to the tank frame 25 and rear frame 35. As viewed from a side of the vehicle 100, the base section 27 partially covers a lower part of the fuel tank 30. As viewed from the side of the vehicle 100, the base section 27 partially covers a front part of the seat 19.

On the right part and left part of the base section 27, a right pressing section 83R and a left pressing section 83L are provided, respectively (FIG. 12 shows only the right pressing section 83R). The right pressing section 83R and left pressing section 83L are supported by the base section 27. The right pressing section 83R and left pressing section 83L are formed to extend rearward and upward from the base section 27.

According to this configuration, the right pressing section 83R and left pressing section 83L extend, respectively, from near the knees of an occupant sitting astride the seat 19 with his/her feet on the steps 28 toward the sides of his/her torso. Namely, the right pressing section 83R and left pressing section 83L are contact units making contact with the right side and left side of the torso of the occupant.

The right pressing section 83R and left pressing section 83L have end parts remote from the base section 27, and the end parts are configured to be movable closer to each other. For instance, an airbag (not shown) may be provided on the left face at a rear end part of the right pressing section 83R and on the right face at an end part of the left pressing section 83L. In this case, the vehicle 100 is provided with a compressor (not shown) as an actuator. The airbags are connected to the compressor through the air pipe. The air pipe is provided with an air valve. Controlling the opening and closing of the air valve adjusts the amount of air to be filled in the airbags on the right pressing section 83R and left pressing section 83L. As a result, the right pressing section 83R and left pressing section 83L are moved closer to each other. The right pressing section 83R and left pressing section 83L approaching each other concurrently press the right side and left side of the occupant's torso.

The mechanism to move the right pressing section 83R and left pressing section 83L closer to each other is not limited to the aforementioned example. For instance, the right pressing section 83R and left pressing section 83L may be configured to swing in the left direction or in the right direction.

The vehicle 100 is equipped with the same control system as that shown in FIG. 7, 8 or 10. The vehicle 100 includes a pressure sensor 41 serving as an approach movement detection unit 42, and a right pressing section 83R and left pressing section 83L serving as two contact units 82. The vehicle 100 also includes a controller 68 and an actuator 6. The actuator 6 applies force, which contains a component that brings the two contact units 82 closer to each other, to at least one of the two contact units 82. The controller 68 controls the force to be applied by the actuator 6 to the contact units 82 based on the pressure detected by the pressure sensor 41.

Therefore, the haptic stimulation application unit 43 can increase the pressure that the right pressing section 83R and left pressing section 83L apply to hold the occupant's torso, in reaction to the action of the occupant holding the grips 16a of the handlebar 16. The increased pressure can make the occupant feel as if he/she is being embraced by the vehicle in sync with the emotional change of the occupant. As a result, the degree of attachment of the occupant to the vehicle can be enhanced.

The configuration of the base section 27 is not limited to the example shown in FIG. 12. For instance, the base section 27 may be attached to the handlebar 16. Alternatively, the fuel tank 30 can be used as the base section, or the base section 27 can be attached to the fuel tank 30. The right pressing section 83R and left pressing section 83L can be formed with components jutting from the base section 27. In addition, the right pressing section 83R and left pressing section 83L can be attached to the cover 26 used as the base section 27.

In FIG. 12, the two contact units, namely the right pressing section 83R and left pressing section 83L, are aligned in the left-right direction; however, the arrangement of the two contact units is not limited thereto. For instance, the two contact units may be arranged in front of and at the rear of the occupant. A front pressing section and a rear pressing section may be disposed in front of and at the rear of the seat 19, respectively. In this case, the seat 19 can be used as a base section. In addition, one of the two contact units can be configured to move. For instance, a pressing section capable of pressing the back of the occupant can be disposed at a rear part of the seat 19. In this case, the force to be applied contains, for example, a directional component that brings the occupant's back abutting against the pressing section closer to the occupant's abdomen abutting against the fuel tank 30.

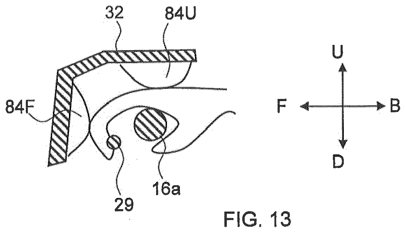

The occupant's body parts with which the contact units make contact are not limited to the occupant's torso. For instance, the contact units may be footrests configured to apply pressure to the ankles or calves of the occupant. In another example, the vehicle can be provided with two contact units that make contact with the occupant's hands. Fig. 13 illustrates a modification of the contact units. FIG. 13 is a cross-sectional view of the vicinity of a grip of the handlebar 16 of the vehicle 100. In the example shown in FIG. 13, a grip cover 32 is provided so as to cover the grip 16a of the handlebar 16 from the front and above. A lever 29 is disposed in front of the grip 16a and at the rear of the grip cover 32. Of the inner face of the grip cover 32, an inner face area in front of the grip 16a and an inner face area above the grip 16a are provided with airbags 84F, 84U, respectively. Specifically, the airbags 84F, 84U are attached at two positions, which are apart from each other in the front-rear direction and up-down direction, in a space between the grip cover 32 and grip 16a.

The airbags 84F, 84U serve as two contact units in contact with different parts of the occupant's hand. The airbags 84F, 84U inflated apply force to the different parts of the occupant's hand from above and the front. These two forces contain components that bring the two contact units closer to each other. Specifically, inflating the airbags 84F, 84U applies force containing a component that brings the two contact units closer to each other in the direction connecting the airbags 84F, 84U, which are the two contact units. This makes the occupant feel as if a hand is laid over the back of the occupant's hand and is holding it. The occupant easily produces a similar feeling to the vehicle 100 to when he/she holds a person's hand, and the person holds his/her hand back.

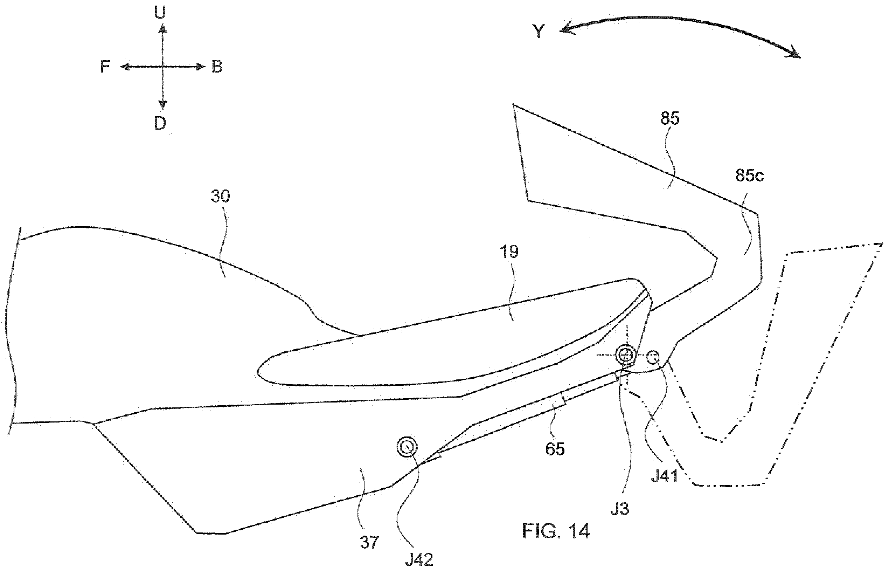

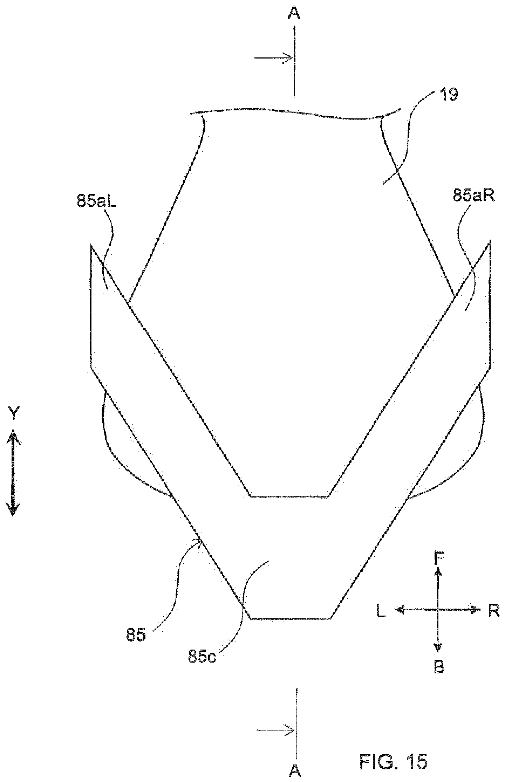

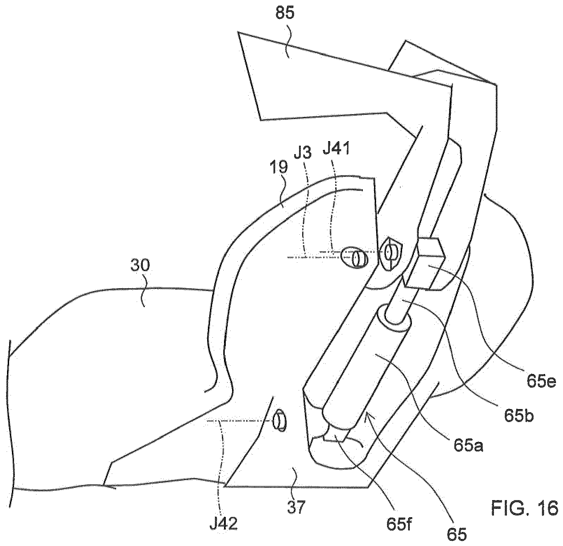

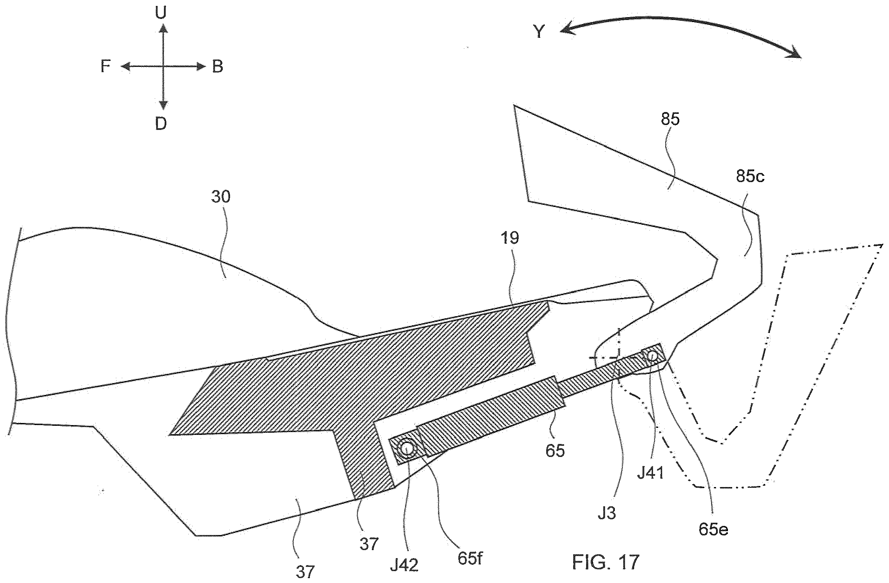

FIGS. 14 to 17 illustrate a modification of the contact units and actuator. FIG. 14 is a left side view showing the configuration of part of a vehicle viewed from the left side of the vehicle. The vehicle's part includes a contact unit 85 and actuator 65 according to the modification. FIG. 15 is a top view of the part of the vehicle, including the contact unit 85 and actuator 65, viewed from above the vehicle. FIG. 16 is a perspective view of the part of the vehicle, including the contact unit 85 and actuator 65, viewed obliquely from the lower left rear of the vehicle. FIG. 17 is a cross-sectional view taken along line A-A in FIG. 15.

As shown in FIG. 14, the contact unit 85 is supported by a body frame 37 so as to turn about a first pivot shaft J3. The body frame 37 has a seat 19 attached thereto. The body frame 37 can be, for example, a rear frame at a rear part of the vehicle. An actuator 65 for turning the contact unit 85 is also attached to the body frame 37. In this example, the actuator 65 is provided opposite to the seat 19 with respect to the body frame 37. In other words, the body frame 37 is disposed between the seat 19 and actuator 65 to support them.

The contact unit 85 is shaped so as to bend toward an occupant sitting on the seat 19. Specifically, the contact unit 85 extends rearward from the first pivot shaft J3, and then bends and extends toward the seat 19. In other words, the contact unit 85 has a bending part 85c that bends toward the seat 19 along the rotational circumferential direction Y of the first pivot shaft J3 which is a rotation axis. The contact unit 85 is configured such that the contact unit 85 turned about the first pivot shaft J3 makes contact with the occupant sitting on the seat 19 from the rear of the vehicle in the front-rear direction. In the example shown in FIG. 14, the axis direction of the first pivot shaft J3 of the contact unit 85 coincides with the left-right direction of the body frame 37. However, the first pivot shaft J3 does not need to coincide with the left-right direction of the body frame 37.

As shown in FIG. 15, the contact unit 85 has a right pressing section 85aR and a left pressing section 85aL. The right pressing section 85aR abuts against the back side of the torso, or the right side of the back, of the occupant sitting on the seat 19. The left pressing section 85aL abuts against the back side of the torso, or the left side of the back, of the occupant. In the example shown in FIG. 15, the right pressing section 85aR and left pressing section 85aL are formed at an end part of the contact unit 85 closer to its tip than the bending part 85c. The contact unit 85 is branched off into the right pressing section 85aR and left pressing section 85aL at a bending part 85c. The right pressing section 85aR and left pressing section 85aL extend separately from the bending part 85c toward the seat 19 in the rotational circumferential direction Y around the first pivot shaft J3.

The face of the right pressing section 85aR with which the occupant makes contact is not parallel with the face of the left pressing section 85aL with which the occupant makes contact. The face of the right pressing section 85aR contacting the occupant is opposed in the left-right direction of the vehicle to the face of the left pressing section 85aL contacting the occupant. When viewed in the left-right direction of the vehicle, at least a part of the right pressing section 85aR overlaps at least a part of the left pressing section 85aL. In the example shown in FIG. 15, the face of the right pressing section 85aR facing the occupant and the face of the left pressing section 85aL facing the occupant are both flat faces. However, these faces may be curved faces.

The contact unit 85 is moved by the actuator 65 to press the right pressing section 85aR and left pressing section 85aL against the occupant in one direction. In this example, the direction in which the right pressing section 85aR and left pressing section 85aL are pressed against the occupant coincides with the circumferential direction Y along which the contact unit 85 turns about the first pivot shaft J3. At this point, a line perpendicular to the face of the right pressing section 85aR contacting the occupant and a line perpendicular to the face of the left pressing section 85aL contacting the occupant are inclined in the left direction of the vehicle or in the right direction of the vehicle with respect to the direction in which the right pressing section 85aR and left pressing section 85aL are pressed against the occupant. The interval between the right pressing section 85aR and left pressing section 85aL decreases with distance from the occupant in the direction in which the pressing sections are pressed against the occupant (circumferential direction Y). More specifically, the right pressing section 85aR and left pressing section 85aL are formed such that the space between the right pressing section 85aR and left pressing section 85aL narrows with distance from the occupant in the direction in which the right pressing section 85aR and left pressing section 85aL are pressed against the occupant. According to the configuration, when the right pressing section 85aR and left pressing section 85aL are pressed against the occupant in one direction, force, which contains a component that brings part of the right pressing section 85aR contacting the occupant and part of the left pressing section 85aL contacting the occupant closer to each other, acts on the occupant. Therefore, the contact unit 85 moved by the actuator 65 can apply force to an occupant's body part sandwiched between the right pressing section 85aR and left pressing section 85aL.

In the example shown in FIG. 15, the right pressing section 85aR and left pressing section 85aL are collectively moved by a single actuator 65. Alternatively, the right pressing section and left pressing section may be configured to be independently moved by an actuator.

As shown in FIGS. 16 and 17, the actuator 65 is connected between the contact unit 85 and the body frame 37. The actuator 65 has a first end 65e that is connected to the contact unit 85 so as to turn about a second pivot shaft J41. The first end 65e of the actuator 65 is connected at a different position from the first pivot shaft J3 of the contact unit 85. The actuator 65 has a second end 65f that is connected to the body frame 37 so as to turn about the third pivot shaft J42. The second pivot shaft J41 and third pivot shaft J42 are coaxial with the first pivot shaft J3. The actuator 65 is made telescopic in the direction connecting the first end 65e and the second end 65f. The actuator 65 turns the contact unit 85 about the first pivot shaft J3 by extending and contracting itself between the first end 65e and second end 65f.

In the example shown in FIGS. 16 and 17, the actuator 65 is an oil hydraulic cylinder. The actuator 65 includes a cylinder 65a and a piston 65b inserted into the cylinder 65a. The piston 65b is movable in the cylinder 65a in the insertion direction. The piston 65b is integrally formed with the first end 65e. The cylinder 65a is integrally formed with the second end 65f. The actuator 65 is configured to be telescopic between the first end 65e and the second end 65f. The actuator 65 may include a driving unit (not shown) that controls the oil pressure in the cylinder 65a. The driving unit can be, for example, an oil-hydraulic pump using a motor as a driving source. In this case, the telescopic motion of the actuator 65 between the first end 65e and second end 65f can be controlled by controlling the output of the motor. Accordingly, the turning movement of the contact unit 85 about the first pivot shaft J3 can be controlled. This means that the pressing force applied by the right pressing section 85aR and left pressing section 85aL of the contact unit 85 to the occupant can be controlled. Thus, the actuator 65 can apply force that contains a component bringing part of the right pressing section 85aR contacting the occupant and part of the left pressing section 85aL contacting the occupant closer to each other.

As described above, the right pressing section 85aR and left pressing section 85aL are located such that the pressing sections applied with force by the actuator 65 can abut against the occupant sitting astride the seat 19 from the rear in the front-rear direction of the vehicle. In this example, the right pressing section 85aR and left pressing section 85aL are located such that the pressing sections abut against the occupant sitting astride the seat 19 from the opposite side of the handlebar (not shown).

The configuration of the actuator 65 is not limited to the example shown in FIGS. 16 and 17. The actuator may include, for example, a motor, and a speed reducer that transfers the rotation of the motor. In this case, the contact unit 85 can be configured to turn about the first pivot shaft J3 with rotation of the speed reducer. In addition, the form of the contact unit located so as to abut against the occupant from the rear is not limited to the example shown in FIGS. 14 to 17.

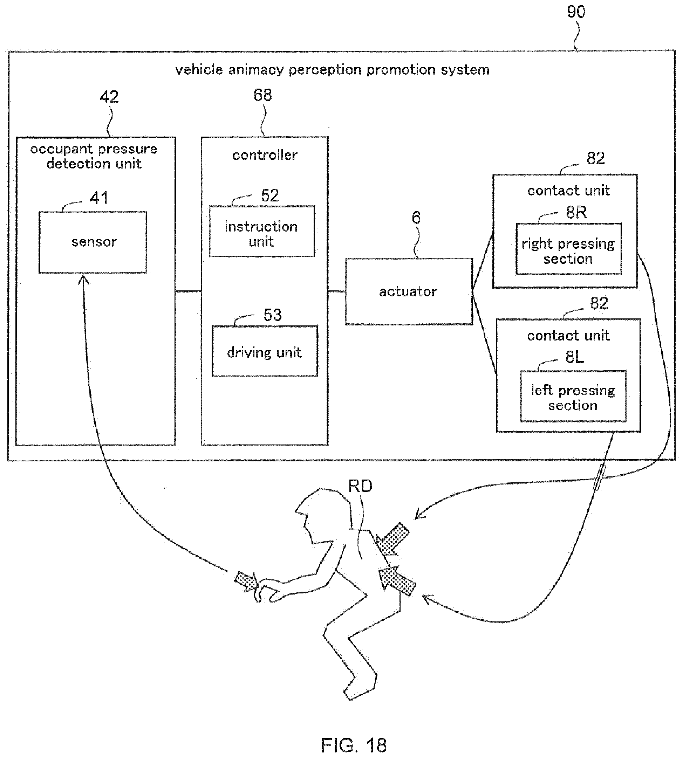

The above-described embodiments including the modifications can be considered as an example of a vehicle animacy perception promotion system. FIG. 18 is a block diagram showing a configuration example of the vehicle animacy perception promotion system by which the above-described embodiments are implemented. The vehicle animacy perception promotion system 90 shown in FIG. 18 includes an occupant pressure detection unit 42, an instruction unit 52, a driving unit 53, an actuator 6, and two contact units 82. The occupant pressure detection unit 42 detects the action of an occupant RD on a vehicle to pressurize the vehicle using his/her muscle strength. The two contact units 82 make contact with different body parts of the occupant RD. The actuator 6 applies force, which contains a component that brings the two contact units 82 closer to each other in the direction connecting the two contact units 82, to at least one of the two contact units 82. The two contact units 82 applied with the force by the actuator 6 can apply pressure to body parts of the occupant RD so as to sandwich a body part of the occupant RD between the two contact units 82. The instruction unit 52 determines the force to be applied by the actuator 6 to at least one of the two contact units 82, in order to promote the occupant RD to perceive animacy in the vehicle, based on the action of the occupant RD detected by the occupant pressure detection unit 42. Upon receipt of an instruction indicating the determined force issued by the instruction unit 52 from the instruction unit 52, the driving unit 53 drives the actuator 6. The actuator 6 driven by the driving unit 53 applies the force, which was determined by the instruction unit 52, to at least one of the two contact units 82.

The occupant pressure detection unit 42 can be a sensor 41 detecting action of the occupant RD pressurizing the vehicle. The occupant pressure detection unit 42 detects action made by the occupant RD to the vehicle, for example, gripping, grasping, embracing, sandwiching, caressing, pushing, pulling, lifting, or dragging down. Detecting the action of the occupant RD can detect the emotional change of the occupant RD. By means of the instruction unit 52, driving unit 53, and actuator 6, the two contact units 82 apply force to body parts of the occupant RD based on the action detected by the occupant pressure detection unit 42. The actuator 6 applies force, which contains a component that brings the two contact units closer to each other in the direction connecting the two contact units making contact with the different body parts of the occupant RD, to at least one of the two contact units. At this point, the two contact units apply pressure to the body parts of the occupant so as to sandwich a body part of the occupant between the two contact units. The instruction unit 52 determines the force to be applied to the occupant RD through the two contact units 82 in response to the action of the occupant RD detected by the occupant pressure detection unit 42. The driving unit 53 receives an instruction from the instruction unit 52, and drives the actuator 6. The occupant RD can feel as if the vehicle returned force to sandwich a body part of the occupant RD between the two parts in reaction to the action of the occupant RD applying pressure to the vehicle. At this moment, the occupant RD can feel as if he/she was wrapped, embraced, clutched, or sandwiched by the vehicle at two separate body parts. Thus, the occupant RD can feel as if he/she was wrapped or embraced by the vehicle in sync with his/her emotional change. In short, this configuration can make the occupant RD recognize animacy in the vehicle. As a result, the psychological state or physiological state of the occupant RD can be changed.

### (Experiment Results)

The inventors studied changes in psychological state or physiological state of occupants RD caused by the vehicle animacy perception promotion system. Subjects participated in driving simulations using a first motorcycle equipped with the contact units 85 and actuator 65 shown in FIGS. 14 to 17, and a second motorcycle without the contact units and actuator. In the driving simulations with the first motorcycle, the actuator was driven such that the contact units 85 apply force to the subjects in response to the action of the subjects applying pressure to the vehicle. The first motorcycle included an auditory stimulation application unit. In the driving simulations with the first motorcycle, the first motorcycle output an exclamation sound with an engine sound in response to the actions of the subjects. Then, endocrine changes of the subjects, more specifically, changes in hormone level of the subjects were measured after the driving simulations. In addition, a survey was conducted using questionnaires to collect the subjects' feedback about the respective driving simulations. The measurement results showed that a hormone (Cortisol) indicating the stress level of the subjects was secreted less in the driving simulations with the first motorcycle than in the driving simulations with the second motorcycle. The measurement results also showed that the hormones (Testosterone and DHEA, which is a precursor to Testosterone) indicating the levels of aggression and competitiveness of the subjects were secreted less in the driving simulations with the first motorcycle than in the driving simulations with the second motorcycle. These hormone measurement results show that the physiological state of the subjects underwent a greater change through the driving simulations with the first motorcycle in comparison with the driving simulations with the second motorcycle. According to the questionnaire results, some subjects felt that the first motorcycle was conscious and cogitative, some felt that the first motorcycle had a human touch, and some felt as if the first motorcycle had emotions. These questionnaire results show that the psychological state of the subjects underwent a greater change through the driving simulations with the first motorcycle in comparison with the driving simulations with the second motorcycle.

### (System Configuration Examples)

In the above-described embodiments, the occupant pressure detection unit, instruction unit, driving unit, actuator, and contact units of the vehicle animacy perception promotion system are installed in a vehicle. However, at least one of the occupant pressure detection unit, instruction unit, driving unit, actuator, and contact units may be provided to a wearable item of the occupant. The wearable item is something worn on the body of the occupant. Examples of the wearable item include a protector, clothing such as a jacket, a helmet, gloves, and shoes. For instance, at least one of the occupant pressure detection unit and contact units may be installed in the wearable item. In addition, the contact units, actuator and driving unit may be installed in a wearable part. Alternatively, the wearable item may be provided with the occupant pressure detection unit, instruction unit, driving unit, actuator, and contact units.

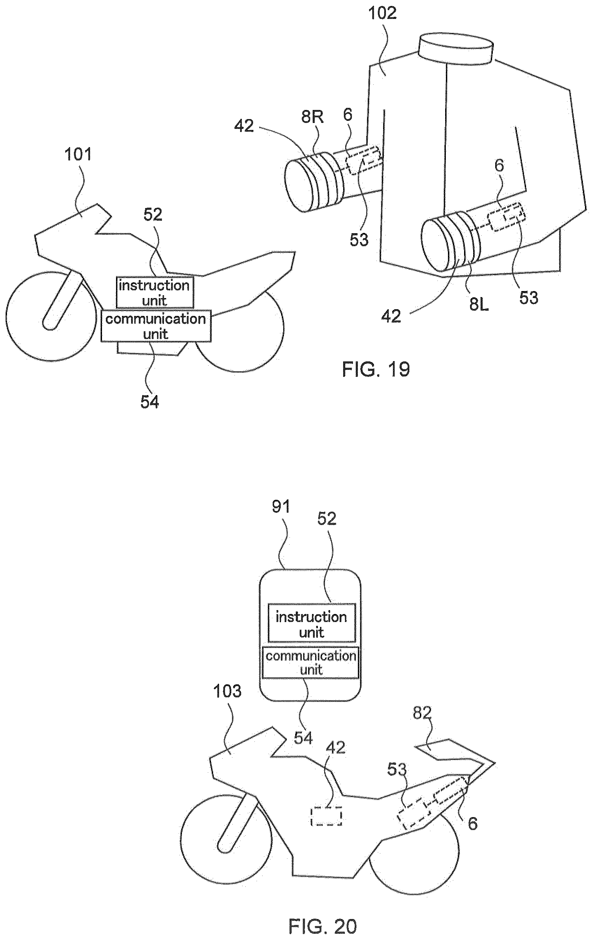

FIG. 19 is a diagram showing a modification of the vehicle animacy perception promotion system. In the modification shown in FIG. 19, a wearable item 102 includes an occupant pressure detection unit 42, a right pressing section 8R and left pressing section 8L serving as contact units, an actuator 6, and a driving unit 53. The wearable item 102 of the modification in FIG. 19 is a jacket. The occupant pressure detection unit 42 is, for example, a pressure sensor or a muscle strength sensor provided inside the sleeves of the jacket. The right pressing section 8R and left pressing section 8L are, for example, airbags provided inside the sleeves of the jacket. In this case, the actuator 6 is a pump that blows air into the airbags. The driving unit 53 is a circuit or a computer that drives the pump. A vehicle 101 is equipped with an instruction unit 52 and a communication unit 54. The communication unit 54 communicates with the occupant pressure detection unit 42 and driving unit 53. The communication unit 54 can include, for example, a receiving unit that receives data or signals indicating a detection result from the occupant pressure detection unit 42, and a transmitting unit that transmits instruction signals or instruction data to the driving unit 53. Incidentally, in FIG. 19, the occupant pressure detection unit 42 may be provided to gloves separate from the jacket. The actuator 6 and driving unit 53 may be installed in the vehicle 101. In addition, the instruction unit 52 can be installed in the wearable item 102.

The instruction unit can be mounted on a terminal device independent from both the vehicle and wearable item. The terminal device can be, for example, a device with a built-in computer. The terminal device may have a communication unit to communicate with the occupant pressure detection unit and driving unit.

FIG. 20 is a diagram showing a modification of the vehicle animacy perception promotion system. In the modification shown in FIG. 20, a vehicle 103 includes an occupant pressure detection unit 42, contact units 82, an actuator 6, and a driving unit 53. A terminal device 91 provided separately from the vehicle 103 includes an instruction unit 52 and a communication unit 54. The terminal device 91 may be, for example, a mobile terminal, such as a smartphone, a tablet PC, a mobile phone, and a PDA. The terminal device 91 executes programs stored in a memory to perform operations of the instruction unit 52. The program that functions the terminal device 91 as the instruction unit 52, and the recording medium storing the program are one of the embodiments of the present teaching. In the configuration shown in FIG. 20, at least one of the occupant pressure detection unit 42, contact units 82, actuator 6, and driving unit 53 can be provided to the wearable item.

### (Other Modifications)

The above-described embodiments are all examples of a leaning vehicle to which the teaching is applied. The leaning vehicle is a vehicle whose body frame leans in the turning direction as it turns. The occupant on the leaning vehicle operates the vehicle using his/her whole body. Therefore, the present teaching capable of increasing attachment of the occupant to the vehicle can be preferably applied to the leaning vehicle. The present teaching can be also applied to straddled vehicles other than the leaning vehicle. In addition, the present teaching can be applied to other types of vehicles than the straddled vehicles.

### (Approach Movement Detection Unit)

The approach movement detection unit does not limit its application to detection of approach movement of fingers and a palm. The approach movement detection unit can be configured to detect movement of other body parts, which are apart from each other, approaching each other. For instance, the approach movement detection unit can be a pressure sensor mounted where the occupant grips the straddled vehicle with knees. In this case, the approach movement detection unit can detect the movement of the right knee and left knee approaching each other.

The sensor used in the approach movement detection unit is also not limited to a specific sensor. The approach movement detection unit can include, for example, a pressure sensor for measuring pressure applied by an occupant's body to the vehicle, an image capturing device for capturing images of the occupant as a subject, a position sensor for detecting the position of the occupant's body parts, or an electromyography sensor for detecting the motion of muscles (electrical activity of muscles) of the occupant. Thus, the approach movement detection unit can detect the movement of the occupant's body parts, which are apart from each other, approaching each other by detecting the pressure applied by the occupant to the vehicle, the image of the occupant as a subject, the positions of the occupant's body parts, or the motion of the occupant's muscles.

The occupant includes a driver and a passenger (who does not drive the vehicle). In the above-described example, the approach movement detection unit detects the movement of a driver, while the contact units make contact with the driver's body. On the contrary, at least one of the occupants to be detected by the approach movement detection unit and to be touched by the contact units may be an occupant other than the driver.

### (Haptic Stimulation Application Unit)

The contact units of the haptic stimulation application unit are part of a vehicle with which the occupant makes contact, including, for example, a seat belt, a seat, a tank, a handle cover, a palm rest, an armrest, a footrest, a step, a shift/accelerator lever, and an accelerator/brake pedal. The form of the force to be applied by the haptic stimulation application unit to the contact units in contact with the occupant includes vibration.

The two contact units can be configured to abut against the occupant's feet, shins, knees, thighs, lower back, hips, chest, shoulders, neck, head, face, upper arms, elbows, or front arms, or any combinations thereof other than the occupant's torso.

The haptic stimulation application unit can apply force in accordance with the amount of the movement (displacement or magnitude of the force) detected by the approach movement detection unit, to the occupant's body parts abutting against the vehicle.

The manner in which the controller of the haptic stimulation application unit controls the force to be applied by the actuator in accordance with the movement detected by the approach movement detection unit can include, for example, a process of determining at least one of the following: the application of force (whether application of force is required or not required), the magnitude of the force, and the timing to apply the force. In short, the controller can control at least one of the application of the force (whether application of force is required or not required), the magnitude of the force, and the timing to apply the force, in accordance with the movement detected by the approach movement detection unit.

### (Controller)

The controller may be configured not only to directly control the actuator, but also indirectly adjust the force to be applied by the actuator.

The controller may control the force to be applied by the actuator based on, in addition to the movement detected by the approach movement detection unit, information about the state of the vehicle. The information about the state of the vehicle can be a physical quantity detected in the vehicle. The physical quantity may be a physical quantity corresponding to the vehicle's state derived from the occupant's action. Cited as examples of the physical quantity are vehicle speed, an amount of leaning or speed of the body frame leaning in the left direction or in the right direction, a throttle opening degree, an amount of brake operation, or a slip ratio.

The controller may be a computer including a processor and a memory. The controller may be a specifically designed circuit. The controller may be made up with a computer and a specifically designed circuit. The controller may include an instruction unit and a driving unit. The instruction unit and driving unit may be made up with at least one of a computer and a circuit. For instance, the operations of the instruction unit may be implemented by a processor executing a program stored in a memory. The operations of the instruction unit may be implemented by at least one of the processor and specifically designed circuit. The operations of the driving unit may be implemented by at least one of the processor and specifically designed circuit. The driving unit outputs control signals to the actuator based on the information supplied by the instruction unit. The driving unit supplies control information to the actuator, while acquiring information indicating the state of the actuator from the actuator. The driving unit has an interface to transmit and receive signals or data to/from the actuator.

### (Exclamation Sound)

The exclamation sounds output by the auditory stimulation application unit are voices expressing human emotions, and the exclamation sounds are not limited to language. The exclamation sounds express human emotions by changing, for example, the pitch or volume of breath sounds. The human emotions expressed by the exclamation sounds are understandable to native speakers of any language. The exclamation sounds are human voices uttering words (sentences) devoid of significance. Examples of the exclamation sounds include a scream, a cry, a gasp, a sigh, or a whistle.

The sounds output by the auditory stimulation application unit are not limited to the exclamation sounds. The auditory stimulation application unit may output a recorded human voice or synthesized human voice. The sounds output by the auditory stimulation application unit may be pseudo voice made in the similitude of a human voice. As described above, the auditory stimulation application unit can output a sound containing a human voice feature amount based on the action of the occupant applying pressure to the vehicle, which is detected by the approach movement detection unit. For instance, detection of the action of the occupant applying pressure to the vehicle by the approach movement detection unit can trigger the auditory stimulation application unit to output a sound containing a voice feature amount. This configuration allows a sound containing a voice feature amount to be output as if to respond to the occupant's action. In addition, the auditory stimulation application unit may determine the voice feature amount in accordance with the time variation of the action of the occupant applying pressure to the vehicle, which is detected by the approach movement detection unit. This configuration enables output of a sound containing a voice feature amount corresponding to the occupant's action. The auditory stimulation application unit may output a sound containing a voice feature amount, for example, while the power source of the vehicle, such as an engine and motor, is in operation. This configuration allows a sound containing a voice feature amount to be output with a sound output by the power source of the vehicle.

### (Vehicle)

The vehicle to which the present teaching is applied can be a transportation machine having two wheels, three wheels, four wheels, or any other number of wheels, and being capable of carrying humans. In addition to the machine with wheels, other types of transportation machines traveling with passengers thereon, such as snowmobiles, watercraft, and aircraft, are also included in the vehicle according to the present teaching. The vehicle to which the present teaching is applied also includes transportation machines propelled by human power, such as bicycles and wheelchairs, in addition to transportation machines propelled by a power source, such as an engine and motor. Also, the present teaching can be applied to vehicles having no seats, for example, vehicles operated by a standing occupant, such as two-wheel electric standing vehicles, and vehicles carrying occupants lying down thereon, such as strollers.

### (Occupant Pressure Detection Unit)

The occupant pressure detection unit detects action of an occupant, who is on a vehicle, applying pressure to the vehicle using his/her muscle strength. The occupant pressure detection unit may be configured to detect pressure applied by a body part of the occupant to a component of the vehicle. The occupant pressure detection unit may directly detect pressure, or may detect other physical quantities related to pressure. For instance, the occupant pressure detection unit may detect a physical quantity indicating a body movement of the occupant on the vehicle. In this case, the occupant pressure detection unit can serve equally as an approach movement detection unit that detects movement of the occupant's body parts, which are apart from each other, approaching each other. In addition, the occupant pressure detection unit may detect a physical quantity related to changes in state of a component in the vehicle. The state of the component is changed by action of the occupant on the vehicle. For instance, the action of the occupant applying pressure to the vehicle can be detected by detecting the physical quantity related to the movement of a movable component of the vehicle, such as a lever, a handlebar, a pedal, a switch, and a grip provided to the vehicle. For instance, the occupant pressure detection unit can detect the action of the occupant's hand or fingers applying pressure to a lever or a throttle by detecting the movement of a brake lever, a clutch lever, or a thumb throttle.

The occupant pressure detection unit detects pressure generated when at least one part of the occupant's body pushes the vehicle. For instance, the occupant pressure detection unit may detect pressure applied in the direction in which two body parts of the occupant abutting against the vehicle approach each other, or may detect action of one body part of the occupant pushing the vehicle. For instance, when the occupant grips the tank with his/her knees, the occupant pressure detection unit may detect action of his/her left knee pushing the tank with his/her right knee being away from a surface of the vehicle as action of the occupant applying pressure to the vehicle.

### (Instruction Unit)

The instruction unit determines the force to be applied by the actuator to at least one of the two contact units based on the occupant's action detected by the occupant pressure detection unit in order to promote the occupant to perceive animacy in the vehicle.

The instruction unit instructs the driving unit such that the actuator applies the determined force to at least one of the two contact units. The instruction unit may output to the driving unit information indicating the way the actuator applies the force to the driving unit. For instance, the instruction unit can transmit to the driving unit an instruction signal or instruction data instructing application of force by the actuator. The driving unit drives the actuator in accordance with the instruction from the instruction unit.

This configuration allows the actuator to move the two contact units in reaction to the action of the occupant applying pressure to the vehicle. As a result, the vehicle can return pressure to the occupant through the two contact units in response to the action of the occupant applying pressure to the vehicle. The two contact units sandwich a body part of the occupant to apply pressure to the body part. This promotes the occupant to perceive animacy in the vehicle.

In an example of the determination process, detection of the occupant's action by the occupant pressure detection unit may trigger the instruction unit to determine the force to be applied by the actuator to at least one of the two contact units. This configuration allows the actuator to apply the force in response to the detected occupant's action. The occupant feels the reaction of the vehicle toward his/her action. Consequently, the occupant is helped to perceive animacy in the vehicle.

In response to the detected occupant's action, the instruction unit determines the force to be applied to the occupant's body part sandwiched between the two contact units driven by the actuator. The force determined by the instruction unit herein may include time variation of force to be applied to at least one of the two contact units. For instance, the instruction unit may determine the timing at which the force is applied or the time-varying pattern of the force to be applied. As described above, the force can be applied in a variety of forms to the occupant's body in response to the occupant's action by the instruction unit determining the time variation of the force to be applied. As a result, the occupant is further helped to perceive animacy in the vehicle.

The instruction unit may determine the force to be applied by the actuator to at least one of the two contact units based on the time variation of the occupant's action detected by the occupant pressure detection unit. Specifically, the instruction unit may determine the force to be applied using information indicating the time variation of the action of the occupant applying pressure to the vehicle. The instruction unit may determine the force corresponding to the action varying over time as force to be applied by the actuator to at least one of the two contact units. The instruction unit can determine the force corresponding to the detected occupant's action varying over time by referring to, for example, data or a program defining the correlation between the time variation of the detected occupant's action and the force to be applied. The force to be applied may be determined using information indicating time variation of the occupant's action, that is, information indicating time variation of physical quantity related to the action of the occupant applying pressure to the vehicle, such as time-series data of physical quantities and waveforms of signals indicating physical quantities. In addition, the information indicating the time variation of the occupant's action used by the instruction unit may include, for example, a duration of the action of the occupant applying pressure to the vehicle, and a time-varying pattern of the occupant's action.

As described above, the instruction unit determines the force to be applied by the actuator to at least one of the two contact units based on the time variation of the detected occupant's action, thereby determining the force to be applied in consideration of a wide variety of the occupant's actions. Thus, this configuration can further promote the occupant's perception of animacy in the vehicle.

In an example of the determination process, the instruction unit may determine at least one of the following based on the action of the occupant detected by the occupant pressure detection unit: the timing at which the actuator applies force to at least one of the two contact units; the magnitude of the force to be applied; and the time-varying pattern of the force to be applied. In other words, the instruction unit can control at least one of the timing at which the contact units return the force to the occupant, the magnitude of the force to be returned, and the time-varying pattern of the force to be applied, in response to the action of the occupant applying pressure to the vehicle. This can enhance the effect of promoting the occupant to perceive animacy in the vehicle.

In the case where the instruction unit determines the timing at which the actuator applies force to at least one of the two contact units, the instruction unit may control the timing to issue an instruction to the driving unit in accordance with the determined timing, or may transmit information indicating the determined timing to the driving unit.

The following are example forms when the instruction unit determines the timing at which the actuator applies force to at least one of the two contact units.

The instruction unit can make an instruction to drive the actuator to apply force to at least one of the two contact units almost at the same time as the occupant pressure detection unit detects the action of the occupant applying pressure to the vehicle. In this case, the instruction unit issues an instruction to the driving unit immediately after the occupant pressure detection unit detects the occupant's action, without waiting. According to the configuration, the action of the occupant applying pressure to the vehicle and the action of the contact units applying force to the occupant can occur almost simultaneously.

The instruction unit can control the time from when the occupant pressure detection unit detects the action of the occupant applying pressure to the vehicle to when the actuator applies force to at least one of the two contact units. For instance, when the occupant pressure detection unit detects an occupant's action, the instruction unit waits for a certain period of time, and then issues an instruction to the driving unit. This can introduce a time lag between the action of the occupant applying pressure to the vehicle and the action of the contact units applying force to the occupant. In addition, the instruction unit can control the length of the time lag by determining the standby time. The instruction unit may control the length of the time lag based on, for example, the occupant's action detected by the occupant pressure detection unit. An example of the detected occupant's action herein is a physical quantity associated with the pressure applied by the occupant to the vehicle (e.g., the magnitude of the pressure or the time variation of the pressure). Alternatively, the instruction unit may control the length of the time lag based on the detected state of the vehicle. An example of the detected state of the vehicle herein can be a physical quantity associated with the travel state of the vehicle (e.g., the vehicle speed, etc.). Also, the instruction unit may control the time lag so as to keep it always constant.

The following are example forms when the instruction unit determines the magnitude of the force to be applied by the actuator to at least one of the two contact units.

The instruction unit may determine whether or not the actuator applies force to at least one of the two contact units based on the magnitude of the pressure applied by the occupant to the vehicle. In addition, the instruction unit may determine the magnitude of the force to be applied by the actuator to at least one of the two contact units in accordance with the magnitude of the pressure applied by the occupant to the vehicle. In this case, the instruction unit may determine application of pressure, which is almost equivalent to the pressure applied by the occupant, from the two contact units to the occupant. Alternatively, the instruction unit may determine application of pressure, which is greater than the pressure applied by the occupant, from the two contact units to the occupant. Alternatively, the instruction unit may determine application of pressure, which is smaller than the pressure applied by the occupant, from the two contact units to the occupant. In addition, the instruction unit may determine the magnitude of the force to be applied by the actuator to at least one of the two contact units in accordance with the amount of action of the occupant applying pressure to the vehicle.

The following are example forms when the instruction unit determines the time-varying pattern of the force to be applied by the actuator to at least one of the two contact units.

The instruction unit may determine the length of the time period in which the actuator applies force to at least one of the two contact units in accordance with the occupant's action detected by the occupant pressure detection unit. The length of the time period in which the actuator applies force to at least one of the two contact units can be controlled in accordance with, for example, the speed at which the actuator moves at least one of the two contact units. The speed at which the actuator moves at least one of the two contact units may be determined by the instruction unit. The instruction unit can determine the length of the time period in which the actuator applies force to at least one of the two contact units, for example, in accordance with one of the following: the amount of action of the occupant applying pressure to the vehicle detected by the occupant pressure detection unit; the rate of time variation of the action (speed); the time-varying pattern of the action; the magnitude of the pressure; the rate of time variation of the pressure; and the time-varying pattern of the pressure.

The instruction unit may determine the number of times the actuator applies force to at least one of the two contact units. For instance, the instruction unit may determine the number of times the actuator applies force intermittently. This configuration allows the actuator to intermittently apply force to at least one of the two contact units at the number of times instructed by the instruction unit.

The instruction unit may determine the waveform of the force to be applied by the actuator to at least one of the two contact units. For instance, the instruction unit may select a waveform corresponding to the detected occupant's action from a plurality of waveform patterns stored in advance. Alternatively, the instruction unit may determine a parameter indicating the waveform, such as a cycle, amplitude, inclination, and the maximum value, based on the detected occupant's action. This configuration allows the instruction unit to determine the time-varying pattern of force to be applied in various ways.

The instruction unit may determine the time-varying pattern of force to be applied by the actuator to at least one of the two contact units so as to match the time-varying pattern of the detected occupant's action. In this case, the instruction unit can perform a process of identifying the time-varying pattern of the occupant's action detected by the occupant pressure detection unit, and a process of determining the time-varying pattern of the to-be-applied force that matches the identified time-varying pattern of the occupant's action. In this case, the instruction unit can determine the time-varying pattern of the to-be-applied force that matches the time-varying pattern of the detected occupant's action by using data or a program defining the correlation between time-varying patterns of the occupant's action and the corresponding time-varying patterns of the force to be applied.

The aforementioned determination processes performed by the instruction unit can include, for example, a process of inputting data indicating a detection result of the occupant pressure detection unit and outputting information indicating an instruction for the actuator. For instance, the instruction unit can determine the instruction for the actuator corresponding to the input detection result by using data or a program indicating the correlation between detection results from the occupant pressure detection unit and the corresponding instructions for the actuator. Alternatively, the instruction unit may include a logic circuit that inputs data indicating detection results of the occupant pressure detection unit and outputs data about instructions for the actuator.

### (Contact Unit)

The vehicle animacy perception promotion system may include two or more contact units. When the system includes three or more contact units, the actuator can be configured to apply force, which contains a component that brings two contact units out of the three or more contact units closer to each other, to at least one of the two contact units. In addition, the contact units may be, for example, doughnut-shaped airbags covering the entire circumference of a body part of the occupant. In this case, the vehicle animacy perception promotion system includes two or more contact units, in other words, an infinite number of contact units.

### (Two Contact Units Pressing Occupant in the Same Direction)

In the example shown in FIGS. 14 to 17, the two contact units are moved by the actuator, and pressed against the occupant in the same direction. The space between the two contact units narrows with distance from the occupant in the direction in which the two contact units are pressed against the occupant with application of force by the actuator. According to the configuration, application of the force by the actuator to the two contact units to move them in one direction produces force containing a component that brings parts of the two contact units contacting the occupant closer to each other. Therefore, the two contact units apply pressure to an occupant's body part located between the two contact units while sandwiching the body part.

## Claims

1. A vehicle animacy perception promotion system comprising:
an occupant pressure detection unit detecting action of an occupant, who is on a vehicle, applying pressure to the vehicle using his/her muscle strength;
two contact units making contact with different body parts of the occupant;
an actuator applying force to at least one of the two contact units such that the two contact units sandwich a body part of the occupant located between the two contact units to apply pressure to the body part, the force containing a component that brings the two contact units closer to each other in the direction connecting parts of the two contact units contacting the body part;
an instruction unit determining the force to be applied by the actuator to at least one of the two contact units based on the action detected by the occupant pressure detection unit to promote the occupant to perceive animacy in the vehicle; and
a driving unit driving the actuator upon receipt of an instruction indicating the force determined by the instruction unit from the instruction unit.

2. The vehicle animacy perception promotion system according to claim 1, further comprising
an auditory stimulation application unit outputting a sound containing a voice feature amount based on the occupant's action of applying pressure to the vehicle detected by the occupant pressure detection unit.

3. The vehicle animacy perception promotion system according to claim 1 or 2, wherein
the instruction unit determines the force to be applied by the actuator to at least one of the two contact units based on the amount of the occupant's action of applying pressure to the vehicle detected by the occupant pressure detection unit to promote the occupant to perceive animacy in the vehicle.

4. The vehicle animacy perception promotion system according to any of claims 1 to 3, wherein
the instruction unit adjusts the force to be applied by the actuator to at least one of the two contact units based on vehicle speed.

5. A vehicle comprising:
two contact units making contact with different body parts of an occupant on the vehicle;
an actuator applying force to at least one of the two contact units such that the two contact units sandwich a body part of the occupant located between the two contact units to apply pressure to the body part, the force containing a component that brings the two contact units closer to each other in the direction connecting parts of the two contact units contacting the body part; and
a driving unit driving the actuator upon receipt of an instruction indicating the force from an instruction unit, the force being determined by the instruction unit based on action of the occupant, who is on the vehicle, applying pressure to the vehicle using his/her muscle strength, the action being detected by an occupant pressure detection unit, and the force being applied by the actuator to at least one of the two contact units to promote the occupant to perceive animacy in the vehicle.

6. The vehicle according to claim 5, wherein
the contact units include a right pressing section making contact with the torso or the right leg of the occupant on the vehicle, and a left pressing section making contact with the torso or the left leg of the occupant on the vehicle, the right pressing section and the left pressing section being aligned in the left-right direction of the vehicle,
the actuator applies force, which brings part of the right pressing section contacting the occupant and part of the left pressing section contacting the occupant closer to each other, to at least one of the right pressing section and the left pressing section, and
the driving unit drives the actuator upon receipt of an instruction indicating the force from the instruction unit, the force being determined by the instruction unit based on the action detected by the occupant pressure detection unit and being applied by the actuator to at least one of the right pressing section and the left pressing section to promote the occupant to perceive animacy in the vehicle.

7. The vehicle according to claim 6 comprising:
a body frame;
a handlebar supported so as to turn at a front portion of the body frame in the front-rear direction of the vehicle; and
a seat supported by the body frame and used by the occupant to sit astride, wherein
the right pressing section and the left pressing section are disposed between the handlebar and the seat.

8. The vehicle according to claim 6 comprising:
the body frame;
a handlebar supported so as to turn at a front portion of the body frame in the front-rear direction of the vehicle; and
a seat provided further rearward in the front-rear direction of the vehicle with respect to the handlebar, and used by the occupant to sit astride, wherein
the right pressing section and left pressing section are located so as to make contact with the occupant sitting astride the seat from the rear in the front-rear direction of the vehicle.

9. A vehicle animacy perception promotion program controlling an actuator, wherein
the actuator applies force to at least one of two contact units such that the two contact units, which make contact with different body parts of an occupant on a vehicle, sandwich a body part of the occupant located between the two contact units to apply pressure to the body part, the force containing a component that brings the two contact units closer to each other in the direction connecting parts of the two contact units contacting the body part, and
the vehicle animacy perception promotion program causes a computer to execute the processes of:
acquiring detection results from an occupant pressure detection unit that detects action of an occupant, who is on the vehicle, applying pressure to the vehicle using his/her muscle strength;
determining the force to be applied by the actuator to at least one of the two contact units based on the action detected by the occupant pressure detection unit to promote the occupant to perceive animacy in the vehicle; and
issuing an instruction indicating the determined force to a driving unit that drives the actuator.

10. A method for controlling an actuator to promote vehicle animacy perception, wherein
the actuator applies force to at least one of two contact units such that the two contact units, which make contact with different body parts of an occupant on a vehicle, sandwich a body part of the occupant located between the two contact units to apply pressure to the body part, the force containing a component that brings the two contact units closer to each other in the direction connecting parts of the two contact units contacting the body part, and
the control method comprises the steps of:
detecting, by using an occupant pressure detection unit, action of the occupant, who is on the vehicle, applying pressure to the vehicle using his/her muscle strength;
determining the force to be applied by the actuator to at least one of the two contact units based on the action detected by the occupant pressure detection unit to promote the occupant to perceive animacy in the vehicle; and
applying the force to at least one of the contact units by using the actuator that is driven by a driving unit upon receipt of an instruction indicating the determined force.
